# EUROPEAN PATENT APPLICATION

(11) **EP 1 683 822 A1**
(43) Date of publication of application: **26.07.2006**
(21) Application number: 04792986.4
(22) Date of filing: 20.10.2004
(51) Int. Cl.: C08G 85/00, C08G 73/06, H01L 21/312

(54) **PREPOLYMER, PREPOLYMER COMPOSITION, HIGH MOLECULAR WEIGHT POLYMER HAVING STRUCTURE CONTAINING HOLE AND ELECTRICALLY INSULATING FILM**

(30) Priority: 05.11.2003 JP 2003376049
(71) Applicant: DAICEL CHEMICAL INDUSTRIES, LTD., Sakai-shi, Osaka 590-8501 (JP)
(72) Inventor: TAKARAGI, Akira, Aboshi-ku, Himeji-shi, Hyogo 671-1234 (JP); FUNAKI, Yoshinori, Himeji-shi, Hyogo 671-1213 (JP); HASHIMOTO, Jiichiro, Himeji-shi, Hyogo 671-1262 (JP)
(74) Representative: Grünecker, Kinkeldey, Stockmair & Schwanhäusser Anwaltssozietät
(86) International application number: PCT/JP2004/015870
(87) International publication number: WO 2005/044899

(57) **Abstract**

A prepolymer is a reaction product of a compound A and a compound B, in which the compounds A and B each have two or more functional groups or sets of functional groups in one molecule and are capable of undergoing polymerization as a result of binding of the functional groups or sets of functional groups of one compound with the functional groups or sets of functional groups of the other compound to thereby form a high-molecular-weight polymer with a porous structure. The prepolymer has a weight-average molecular weight of about 200 to about 100000. The functional groups or sets of functional groups of the compound A are preferably each a carboxyl group or an amino group, and the functional groups or sets of functional groups of the compound B are preferably two amino groups, an amino group and a hydroxyl group, an amino group and a mercapto group, or two carboxyl groups.

## Description

### Technical Field

The present invention relates to dielectric films used typically for the production of semiconductors. More specifically, it relates to dielectric films which are excellent in heat resistance and mechanical strength and show low relative dielectric constants, and production methods thereof. It also relates to prepolymers useful for preparing the dielectric films, prepolymer compositions comprising the prepolymers, and high-molecular-weight polymers with a porous structure.

### Background Art

The dielectric constants of interlayer dielectric films of very large scale integrated circuits (VLSI) are believed to be effectively reduced by constructing porous structures, and, for example, the introduction of porous structures into films of silane compounds by typically using a foaming agent has been proposed. Although pores can be formed according to this technique, the resulting films inevitably have combined pores (continuous pores), are thereby insufficient in mechanical strength and thermostability, and face significant problems such as film disruption in interconnection process in the production of semiconductors.

Certain interlayer dielectric films are organic polymer films such as polymer films containing polyimide derivatives, polymer films containing poly(allyl ether derivative)s, and polymer films containing poly(p-xylene derivative)s. Such organic polymer films realize their porous structures by cleaving crosslinking sites in the constitutive organic polymers. However, the cleaving of crosslinking sites invites impaired mechanical strength and decreased heat resistance.

Polybenzazoles having adamantane skeletons are useful as highly thermostable resins (see, for example, "Journal of Polymer Science" Part A-1 (1970), 8(12), p.3665-6). Among them, highly crosslinked polybenzazoles using trifunctional or tetrafunctional adamantanes are very useful as materials for interlayer dielectric films, because they have a multiplicity of molecular-scale pores and thereby show low relative dielectric constants and sufficient mechanical strength and heat resistance (see, for example, Japanese Unexamined Patent Application Publication (JP-A) No. 2001-332543). These highly crosslinked polybenzazoles can be synthetically prepared, for example, by a production process such as heating in the presence of a condensing agent such as a polyphosphoric acid. The resulting highly-crosslinked resins, however, are very insoluble in a solvent, cannot be significantly applied as a thin film to a substrate typically by coating, and films having thicknesses necessary as interlayer dielectric films cannot be significantly formed.

A thin film of a wholly aromatic chain polybenzazole is formed by a certain method including the steps of spreading an aldehyde derivative as a material monomer over an aqueous solution containing an amine monomer as another material monomer; allowing polymerization to proceed to thereby form a film on a gas-liquid interface; laminating formed films on a substrate by a horizontal attachment method; and carrying out heat treatment in the air (see, for example, JP-A No. 62-183881). However, this method is not suitable for industrial production, since it takes quite a long time to form the target thin film. In addition, the precursor polyimine is subjected to an oxidative thermal treatment in a final process, and the resulting polybenzazole film may be possibly oxidized, thus a lower dielectric constant necessary as a dielectric film is not expected.

### Disclosure of Invention

An object of the present invention is to provide high-molecular-weight polymers and dielectric films which have high heat resistance and low relative dielectric constants and are useful for the production of semiconductors, and production methods of them, prepolymers and prepolymer compositions that can form these polymers and dielectric films.

Another object of the present invention is to provide prepolymers and prepolymer compositions that can easily form thin films having necessary thicknesses as interlayer dielectric films and to provide high-molecular-weight polymers and dielectric films formed from these prepolymers and prepolymer compositions.

Yet another object of the present invention is to provide high-molecular-weight polymers and dielectric films that are highly crosslinked and highly porous and to provide prepolymers and prepolymer compositions that can form these polymers and dielectric films.

After intensive investigations to achieve the above objects, the present inventors have found that a dielectric film having a very low relative dielectric constant and a desired thickness can be easily obtained by carrying out the production of a high-molecular-weight polymer with a porous structure in two processes, specifically, by preparing a prepolymer easily soluble in a solvent in advance, and converting the prepolymer into a high-molecular-weight polymer typically by heat treatment. More specifically, they have found that a dielectric film comprising a high-molecular-weight polymer with a porous structure having high heat resistance, high mechanical strength, and a very low relative dielectric constant can be obtained by reacting two compounds under suitable conditions to yield a prepolymer as a precursor of the high-molecular-weight polymer, which two compounds are capable of undergoing polymerization to form a high-molecular-weight polymer with a porous structure as a result of a reaction between their functional groups; dissolving the prepolymer in an appropriate solvent; applying the solution onto a base material; and subjecting the applied film typically to heat treatment to allow reactions such as chain extension, cyclization, and crosslinking to proceed smoothly to thereby form pores reliably. They also have found that the prepolymer is excellent in solubility in a solvent and can thereby easily yield a thin film having a necessary thickness as interlayer dielectric films. The present invention has been achieved based on these findings.

Specifically, the present invention provides a prepolymer as a reaction product between a compound A and a compound B, the compound A and the compound B each having two or more functional groups or sets of functional groups in one molecule, and the compound A and the compound B being capable of undergoing polymerization as a result of binding of the functional groups or sets of functional groups of one compound with the functional groups or sets of functional groups of the other compound to thereby form a high-molecular-weight polymer with a porous structure.

The prepolymer has a weight-average molecular weight of, for example, 200 to 100000.

The prepolymer includes, for example, a prepolymer which is a reaction product of a compound A and a compound B, the compound A and the compound B being capable of forming a high-molecular-weight polymer with a porous structure as a result of a polymerization process, the polymerization process comprising a first reaction process of allowing the functional groups or sets of functional groups of one compound to react with the functional groups or sets of functional groups of the other compound to form a chain bond; and a second reaction process of forming a ring at the binding site, wherein the prepolymer is a reaction product of the first reaction process. Examples of such prepolymers include a prepolymer in which the chain bond formed in the first reaction process is one of an amide bond, an ester bond, and a thioester bond, and the ring which will be formed in the second reaction process is one of an imidazole ring, an oxazole ring, a thiazole ring, and an imide ring, in the reaction between the functional groups or sets of functional groups of the compound A and the functional groups or sets of functional groups of the compound B. Examples of the prepolymer just mentioned above include a prepolymer in which the functional groups or sets of functional groups of the compound A are each a carboxyl group or an amino group, and the functional groups or sets of functional groups of the compound B are two amino groups; an amino group and a hydroxyl group; an amino group and a mercapto group; or two carboxyl groups.

In the prepolymers according to the present invention, the compound A can be a compound represented by following Formula (1): 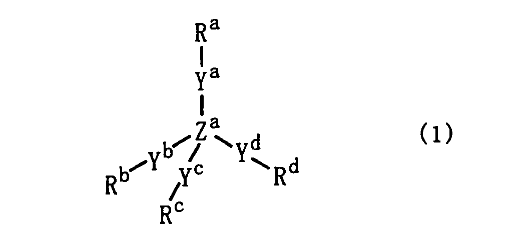 wherein Z^{a} represents a quadrivalent organic group; R^{a}, R^{b}, R^{c}, and R^{d} are the same as or different from one another and each represent a protected or unprotected carboxyl group, a haloformyl group, a protected or unprotected amino group, a protected or unprotected hydroxyl group, a protected or unprotected mercapto group, a hydrogen atom, or a hydrocarbon group; and Y^{a}, Y^{b}, Y^{c}, and Y^{d} are the same as or different from one another and each represent a single bond or a bivalent aromatic or nonaromatic cyclic group, wherein at least two of R^{a}, R^{b}, R^{c}, and R^{d} each represent a protected carboxyl group, a haloformyl group, a protected or unprotected amino group, a protected or unprotected hydroxyl group, or a protected or unprotected mercapto group.

The compound B can be a compound represented by following Formula (2): 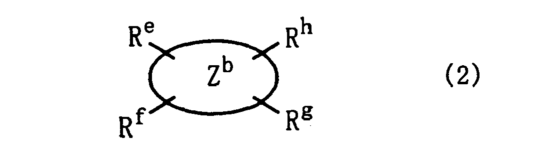 wherein Ring Z^{b} represents a monocyclic or polycyclic aromatic or nonaromatic ring; and R^{e}, R^{f}, R^{g}, and R^{h} are substituents bound to Ring Z^{b}, are the same as or different from one another, and each represent a protected or unprotected amino group, a protected or unprotected hydroxyl group, a protected or unprotected mercapto group, or a protected or unprotected carboxyl group.

The prepolymers according to the present invention also include a prepolymer which is a reaction product between at least one adamantanepolycarboxylic acid derivative represented by following Formula (3), (4), or (5): 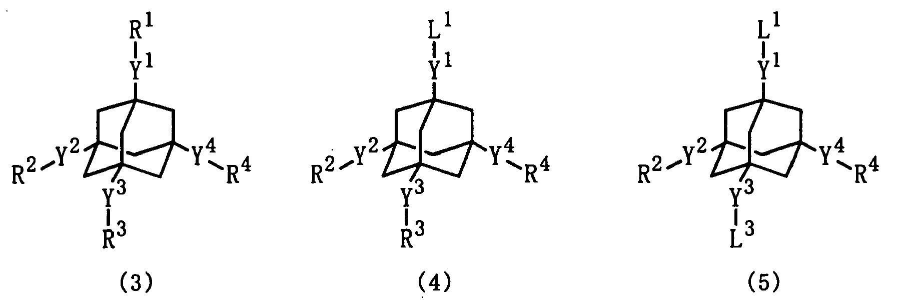 wherein R¹, R², R³, and R⁴ are the same as or different from one another and each represent a protected or unprotected carboxyl group or a haloformyl group; Y¹, Y², Y³, and Y⁴ are the same as or different from one another and each represent a single bond or a bivalent aromatic or nonaromatic cyclic group; and L¹ and L³ are the same as or different from each other and each represent a hydrogen atom, a protected or unprotected carboxyl group, or a hydrocarbon group, and a polyamine derivative represented by following Formula (6) : 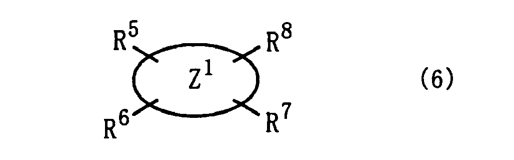 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer comprises an amide bond, an ester bond, or a thioester bond formed as a result of a reaction of the protected or unprotected carboxyl group or haloformyl group in R¹, R², R³, and R⁴ of the adamantanepolycarboxylic acid derivative represented by Formula (3), (4), or (5) with the protected or unprotected amino group, protected or unprotected hydroxyl group, or protected or unprotected mercapto group in R⁵, R⁶, R⁷, and R⁸ of the polyamine derivative represented by Formula (6).

These prepolymers include, as a first embodiment, a prepolymer as a reaction product between an adamantanepolycarboxylic acid derivative represented by following Formula (3): 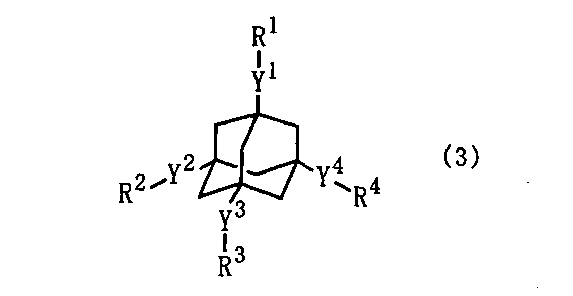 wherein R¹, R², R³, and R⁴ are the same as or different from one another and each represent a protected or unprotected carboxyl group or a haloformyl group; and Y¹, Y², Y³, and Y⁴ are the same as or different from one another and each represent a single bond or a bivalent aromatic or nonaromatic cyclic group,
and a polyamine derivative represented by following Formula (6): 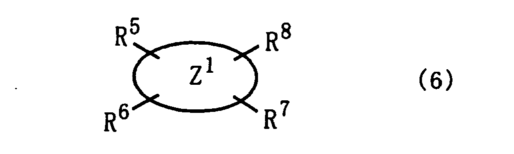 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (7): 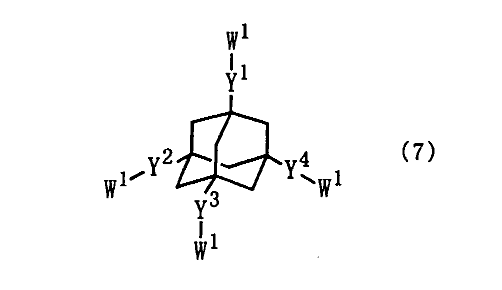 wherein Y¹, Y², Y³, and Y⁴ are as defined above; and W¹s each represent one of R¹, R², R³, R⁴, and a group represented by following Formula (8): 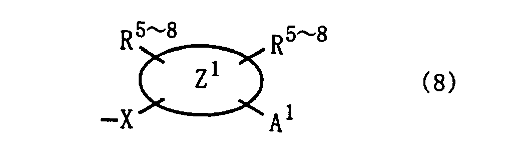 wherein Ring Z¹ is as defined above; R⁵⁻⁸ represents any one of R⁵, R⁶, R⁷, and R⁸; X is a bond formed as a result of a reaction between one of R¹ to R⁴ and one of R⁵ to R⁸ and represents an amide bond, an ester bond, or a thioester bond; and A¹ represents one of R⁵, R⁶, R⁷, R⁸, and a group represented by following Formula (9): 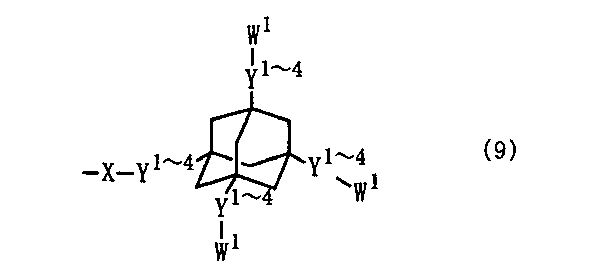 wherein W¹s and X are as defined above; and Y¹⁻⁴ represents any one of Y¹, Y², Y³, and Y⁴,
wherein at least one of the four W¹s in Formula (7) is the group represented by Formula (8), and wherein A¹s, W¹s, and Xs, if present plural in number, may be the same as or different from each other, respectively.

The prepolymers also include, as a second embodiment, a prepolymer as a reaction product between an adamantanepolycarboxylic acid derivative represented by following Formula (4): 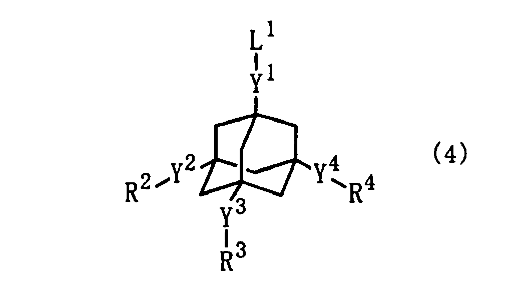 wherein L¹ represents a hydrogen atom, a protected or unprotected carboxyl group, or a hydrocarbon group; R², R³, and R⁴ are the same as or different from one another and each represent a protected or unprotected carboxyl group or a haloformyl group; and Y¹, Y², Y³, and Y⁴ are the same as or different from one another and each represent a single bond or a bivalent aromatic or nonaromatic cyclic group,
and a polyamine derivative represented by following Formula (6): 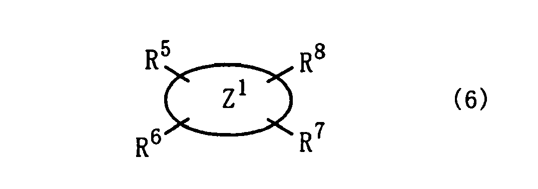 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (10): 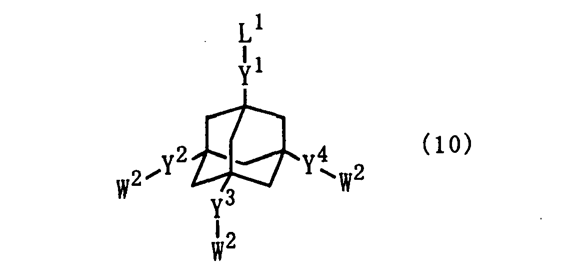 wherein L¹, Y¹, Y², Y³, and Y⁴ are as defined above; and W²s each represent one of R², R³, R⁴, and a group represented by following Formula (11): 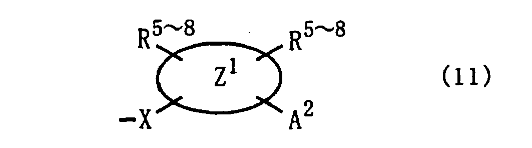 wherein Ring Z¹ is as defined above; R⁵⁻⁸ represents any one of R⁵, R⁶, R⁷, and R⁸; X is a bond formed as a result of a reaction between one of R² to R⁴ and one of R⁵ to R⁸ and represents an amide bond, an ester bond, or a thioester bond; and A² represents one of R⁵, R⁶, R⁷, R⁸, and a group represented by following Formula (12): 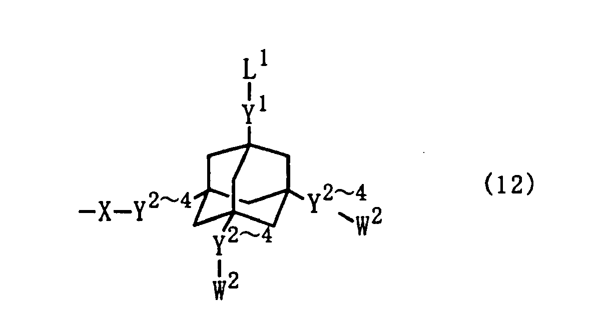 wherein L¹, W²s, and X are as defined above; and Y²⁻⁴ represents any one of Y², Y³, and Y⁴,
wherein at least one of the three W²s in Formula (10) is the group represented by Formula (11), and wherein A²s, W²s, and Xs, if present plural in number, may be the same as or different from each other, respectively.

The prepolymers further include, as a third embodiment, a prepolymer as a reaction product between an adamantanepolycarboxylic acid derivative represented by following Formula (5): 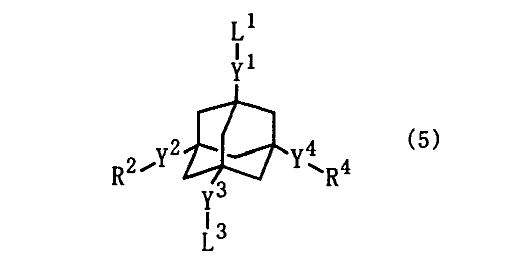 wherein L¹ and L³ are the same as or different from each other and each represent a hydrogen atom, a protected or unprotected carboxyl group, or a hydrocarbon group; R² and R⁴ are the same as or different from each other and each represent a protected or unprotected carboxyl group or a haloformyl group; and Y¹, Y², Y³, and Y⁴ are the same as or different from one another and each represent a single bond or a bivalent aromatic or nonaromatic cyclic group,
and a polyamine derivative represented by following Formula (6): 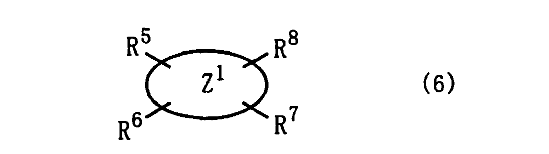 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (13): 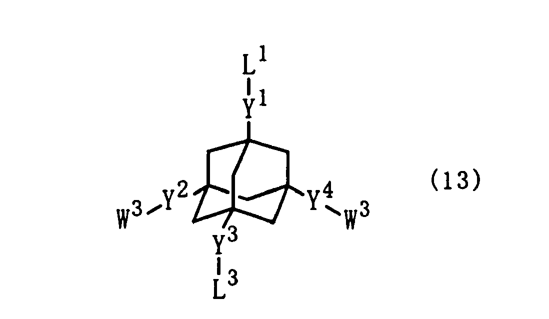 wherein L¹, L³, Y¹, Y², Y³, and Y⁴ are as defined above; and W³s each represent one of R², R⁴, and a group represented by following Formula (14): 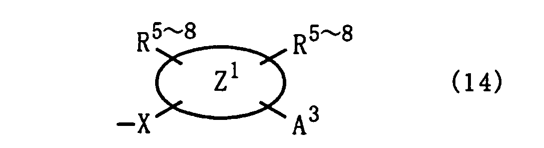 wherein Ring Z¹ is as defined above; R⁵⁻⁸ represents any one of R⁵, R⁶, R⁷, and R⁸; X is a bond formed as a result of a reaction between one of R² and R⁴ and one of R⁵ to R⁸ and represents an amide bond, an ester bond, or a thioester bond; and A³ represents one of R⁵, R⁶, R⁷, R⁸, and a group represented by following Formula (15): 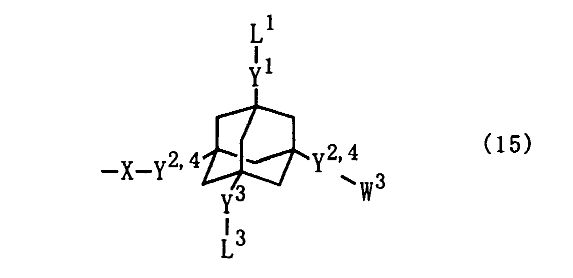 wherein L¹, L³, W³, and X are as defined above; and Y^{2,4} represents one of Y² and Y⁴,
wherein at least one of the two W³s in Formula (13) is the group represented by Formula (14), and wherein A³s, W³s , and Xs, if present plural in number, may be the same as or different from each other, respectively.

The present invention further provides a prepolymer composition, as a solution of the prepolymer in a solvent.

The present invention additionally provides a high-molecular-weight polymer with a porous structure, as a reaction product of the prepolymer.

The present invention further provides a dielectric film comprising the high-molecular-weight polymer with a porous structure

In addition and advantageously, the present invention provides a method for producing a dielectric film, comprising the steps of applying a prepolymer composition to a base material, the prepolymer composition being a solution of the prepolymer in a solvent; and subjecting the prepolymer composition on the base material to reaction to thereby yield a dielectric film comprising a high-molecular-weight polymer with a porous structure.

The prepolymers according to the present invention structurally have moderately polymerized constitutional units (monomer units) of final target high-molecular-weight polymers with a porous structure. When the prepolymers are further reacted, reactions such as polycondensation reactions (chain extension and/or crosslinking) proceed smoothly to yield higher molecular weights and cyclic structures, to thereby yield highly homogenous high-molecular-weight polymers having a multiplicity of pores with desired sizes. The prepolymers using a compound having three or higher functionalities as at least one of materials can yield highly crosslinked high-molecular-weight polymers (crosslinked polymers) having the above-mentioned properties. These can yield, in turn, dielectric films having low dielectric constants, high heat resistance, and high mechanical strength. Additionally, the prepolymers can easily form dielectric films having necessary thicknesses as interlayer dielectric films due to their excellent solubilities in a solvent.

The high-molecular-weight polymers and dielectric films according to the present invention show low relative dielectric constants due to their high porosities and have high heat resistance and high mechanical strength.

### Best Mode for Carrying Out the Invention

The prepolymers according to the present invention are compounds each as a reaction product between a compound A and a compound B and serve as precursors for high-molecular-weight polymers. The compound A and the compound B each have two or more functional groups or sets of functional groups in one molecule. These two compounds constitute such a combination as to undergo polymerization and yield a high-molecular-weight polymer with a porous structure as a result of binding of the functional groups or sets of functional groups of one compound and the functional groups or sets of functional groups of the other compound. In the present description, a compound having two functional groups or sets of functional groups is also referred to as "bifunctional" compound, one having three functional groups or sets of functional groups is also referred to as "trifunctional" compound, and one having four functional groups or sets of functional groups is also referred to as "tetrafunctional" compound.

The central skeletons of the compound A and the compound B can each comprise, for example, carbon atoms, oxygen atoms, silicon atoms, nitrogen atoms, and/or sulfur atoms, and they each generally comprise one hundred or less atoms. The molecular weights of the compound A and the compound B are, for example, 50 to about 1500, and preferably about 100 to about 1000, respectively.

Such combinations of the compounds A and B that can yield a high-molecular-weight polymer with a porous structure include a combination of a compound A in which plural (e.g., two to four) functional groups or sets of functional groups bound to a central skeleton constitute a two-dimensional structure or a three-dimensional structure; with a compound B in which plural (e.g., two to four, preferably two) functional groups or sets of functional groups bound to a central skeleton constitute a one-dimensional (linear) structure or a two-dimensional (straight-chain at an angle) structure. In this case, the compound A constitutes nodal points or crosslinking points (vertices), and the compound B constitutes joining sections (sides) connecting the nodal points or crosslinking points, in the resulting high-molecular-weight polymer. Spaces each surrounded by some nodal points or crosslinking points and some joining sections constitute pores. The high-molecular-weight polymers can be polymers having a branched structure, especially a multiply branched structure, or polymers comprising unbranched chain polymer molecules. Even such polymers comprising unbranched chain polymer molecules constitute a relatively loose packing structure, since one polymer molecular chain is prevented from penetrating a region occupied by another polymer molecule due to the excluded volume effect between segments in the polymer molecular chain. The porous structures also include structures of this type.

In the above case, examples of the central skeleton of the compound A include nonaromatic ring skeletons including bridged ring skeletons, such as adamantane skeleton, tetraphenyladamantane skeleton, norbornane skeleton, tetramethylnorbornane skeleton, norbornene skeleton, and tetramethylnorbornene skeleton; skeletons having a carbon atom at the center, such as tetraphenylmethane skeleton; skeletons having a two-dimensional structure, such as porphyrin skeleton; and chain skeletons including chain hydrocarbon skeletons having about four to about ten carbon atoms, such as butane skeleton. The central skeleton moiety of the compound A has a molecular weight of, for example, about 40 to about 1460. Examples of the central skeleton of the compound B include monocyclic or polycyclic aromatic or nonaromatic ring skeletons such as benzene ring and biphenyl ring.

The functional groups are not specifically limited, as long as they have reactivity, and representative examples thereof include carboxyl group, amino group, hydroxyl group, mercapto group, silanol group, halogen atoms, carbanion, and groups containing these. These groups may be converted into reactive groups and may be protected by a protecting group. Examples of reactive groups derived from, for example, carboxyl group include haloformyl groups and acid anhydride groups. Such acid anhydride groups can also be classified as protected carboxyl groups. The protecting groups can be conventional protecting groups used in the field of organic synthesis.

Examples of such combinations of functional groups or sets of functional groups so as to form a chemical bond as a result of the reaction with each other include, but are not limited to, a combination of a carboxyl group and an amino group to form an amide bond, a combination of a carboxyl group and a hydroxyl group to form an ester bond, a combination of a carboxyl group and a mercapto group to form a thioester bond, a combination of a hydroxyl group and a hydroxyl group to form an ether bond, a combination of a hydroxyl group and a mercapto group to form a thioether bond, a combination of two functional groups that can form a carbon-carbon bond, and a combination of two functional groups that can form a carbon-nitrogen bond; a combination of one carboxyl group with two amino groups bound to carbon atoms at the 1- and 2-positions or 1- and 3-positions to form a five- or six-membered ring having two nitrogen atoms, such as imidazole ring, a combination of one carboxyl group with an amino group and a hydroxyl group bound to carbon atoms at the 1- and 2-positions or 1- and 3-positions to form a five- or six-membered ring having one nitrogen atom and one oxygen atom, such as oxazole ring, a combination of one carboxyl group with one amino group and one mercapto group each bound to carbon atoms at the 1- and 2-positions or 1- and 3-positions to form a five- or six-membered ring having one nitrogen atom and one sulfur atom, such as thiazole ring, and a combination of two carboxyl groups bound to carbon atoms at the 1- and 2-positions or 1- and 3-positions with one amino group to form a five- or six-membered imide ring.

The prepolymers according to the present invention are reaction products that are derived from the compounds A and B and lead to high-molecular-weight polymers with a porous structure. The concept of prepolymers herein includes oligomers having a low molecular weight (degree of polymerization), as well as precursor polymers having precursor structures corresponding to the structures of the ultimate high-molecular-weight polymers. The precursor polymers include, for example, polymers having a chain structure before cyclization in the case where the resulting high-molecular-weight polymers are formed as a result of ring formation by cyclization. The prepolymers each have a weight-average molecular weight of, for example, about 200 to about 100000, preferably about 300 to about 50000, and more preferably about 1000 to about 30000.

The prepolymers can be compounds having a branched structure (especially a multiply branched structure) or unbranched chain compounds. The prepolymers generally have stereo structures identical to or analogous to partial structures of the stereo structures of the resulting high-molecular-weight polymers with a porous structure. High-molecular-weight polymers with a porous structure can be obtained by allowing the prepolymers to have higher molecular weights or subjecting them to crosslinking and/or cyclization.

Preferred examples of the prepolymers according to the present invention include a prepolymer as a reaction product of a compound A and a compound B, the compound A and the compound B being capable of forming a high-molecular-weight polymer with a porous structure as a result of a polymerization process, the polymerization process comprising a first reaction process of allowing the functional groups or sets of functional groups of one compound to react with the functional groups or sets of functional groups of the other compound to form a chain bond; and a second reaction process of forming a ring at the binding site, wherein the prepolymer is a reaction product of the first reaction process. Further cyclization of prepolymers of this type can yield high-molecular-weight polymers having high heat resistance and high mechanical strength in which the central skeletons of the two compounds are combined with each other with the interposition of a ring.

The above-mentioned prepolymers include, for example, prepolymers in which the chain bond formed in the first reaction process is one of an amide bond, an ester bond and a thioester bond, and the ring which will be formed in the second reaction process is one of an imidazole ring, an oxazole ring, a thiazole ring, and an imide ring. Such prepolymers can be prepared by subjecting a compound A and a compound B to a reaction to form an amide, an ester, or a thioester, in which the compound A has a carboxyl group or an amino group as the functional groups or sets of functional groups, and the compound B has two amino groups, an amino group and a hydroxyl group, an amino group and a mercapto group, or two carboxyl groups as the functional groups or sets of functional groups.

Representative examples of the compound A in the prepolymers according to the present invention include the compounds represented by Formula (1). In Formula (1), Z^{a} represents a quadrivalent organic group; R^{a}, R^{b}, R^{c}, and R^{d} are the same as or different from one another and each represent a protected or unprotected carboxyl group, a haloformyl group, a protected or unprotected amino group, a protected or unprotected hydroxyl group, a protected or unprotected mercapto group, a hydrogen atom, or a hydrocarbon group; and Y^{a}, Y^{b}, Y^{c}, and Y^{d} are the same as or different from one another and each represent a single bond or a bivalent aromatic or nonaromatic cyclic group, where at least two of R^{a}, R^{b}, R^{c}, and R^{d} each represent a protected carboxyl group, a haloformyl group, a protected or unprotected amino group, a protected or unprotected hydroxyl group, or a protected or unprotected mercapto group. In this compound, the protected carboxyl group, haloformyl group, protected or unprotected amino group, protected or unprotected hydroxyl group, or protected or unprotected mercapto group corresponds to the functional groups or sets of functional groups.

Examples of the organic group in Z^{a} include quadrivalent aromatic or nonaromatic cyclic groups, quadrivalent acyclic groups, and quadrivalent groups each comprising a plurality of these groups combined with each other. Aromatic rings constituting the quadrivalent aromatic cyclic groups include polycyclic rings, such as porphyrin ring, which comprise a plurality of aromatic carbon rings, such as benzene ring, and/or heteroaromatic rings, such as pyrrole ring, combined with each other with the interposition of one or more linkage groups. Examples of the linkage groups are single bond; bivalent hydrocarbon groups such as methylene group and vinylene group; oxygen atom; nitrogen atom; silicon atom; and sulfur atom. Nonaromatic rings constituting the quadrivalent nonaromatic cyclic groups include monocyclic or polycyclic (bridged) alicyclic carbon rings or nonaromatic heterocyclic rings, such as adamantane ring, norbornane ring, and norbornene ring. Examples of the quadrivalent acyclic groups include carbon atom; and chain hydrocarbon groups such as butane-1,2,3,4-tetrayl group and 2,3-dimethylbutane-1,2,3,4-tetrayl group.

Examples of the "protecting groups" of the protected or unprotected carboxyl groups in R^{a}, R^{b}, R^{c}, and R^{d} include alkoxy groups including alkoxy groups having one to ten carbon atoms, such as methoxy, ethoxy, propoxy, isopropoxy, butoxy, isobutoxy, s-butoxy, t-butoxy, and hexyloxy, and (C₁₋₄ alkoxy) ₁₋₂- (C₁₋₄ alkoxy) groups such as methoxymethyloxy and methoxyethoxymethyloxy groups; cycloalkyloxy groups including cycloalkyloxy groups having three to twenty carbon atoms, such as cyclopentyloxy and cyclohexyloxy; tetrahydrofuranyloxy group; tetrahydropyranyloxy group; aryloxy groups including aryloxy groups having six to twenty carbon atoms, such as phenoxy and methylphenoxy groups; aralkyloxy groups including aralkyloxy groups having seven to eighteen carbon atoms, such as benzyloxy and diphenylmethyloxy groups; trialkylsilyloxy groups including tri(C₁₋₄ alkyl)silyloxy groups such as trimethylsilyloxy and triethylsilyloxy groups; substituted or unsubstituted amino groups including amino group, mono- or di-(C₁₋₆ alkyl) amino groups such as methylamino, dimethylamino, ethylamino, and diethylamino, and cyclic amino groups such as pyrrolidino and piperidino groups; substituted or unsubstituted hydrazino groups including hydrazino group, N-phenylhydrazino group, alkoxycarbonylhydrazino groups such as t-butoxycarbonylhydrazino group and other (C₁₋₁₀ alkoxy)carbonylhydrazino groups, aralkyloxycarbonylhydrazino groups such as benzyloxycarbonylhydrazino group and other (C₇₋₁₈ aralkyl)oxycarbonylhydrazino groups; acyloxy groups including C₁₋₁₀ acyloxy groups such as acetoxy and propionyloxy groups; and acyl groups including acyl groups having one to ten carbon atoms, such as acetyl and propionyl groups. The carboxyl-protecting groups are not limited to these, and other protecting groups used in the field of organic synthesis can also be used.

Preferred examples of protected carboxyl groups include (C₁₋₆ alkoxy) -carbonyl groups, (C₁₋₄ alkoxy) ₁₋₂- (C₁₋₄ alkoxy) - carbonyl groups, N-substituted carbamoyl groups, tetrahydropyranyloxycarbonyl group, tetrahydrofuranyloxycarbonyl group, aryloxycarbonyl groups, and trialkylsilyloxycarbonyl groups.

The haloformyl groups (carbonyl halide groups) in R^{a}, R^{b}, R^{c}, and R^{d} include chloroformyl group (carbonyl chloride group), bromoformyl group (carbonyl bromide group), fluoroformyl group (carbonyl fluoride group), and iodoformyl group (carbonyl iodide group).

Examples of "protecting groups" of the protected or unprotected amino groups in R^{a}, R^{b}, R^{c}, and R^{d} include acyl groups including aliphatic acyl groups having one to six carbon atoms, such as formyl, acetyl, propionyl, butyryl, isobutyryl, valeryl, and pivaloyl groups, and aromatic acyl groups having about six to about twenty carbon atoms, such as benzoyl and naphthoyl groups; alkoxycarbonyl groups including (C₁₋₄ alkoxy)-carbonyl groups such as methoxycarbonyl, ethoxycarbonyl, and t-butoxycarbonyl; aralkyloxy-carbonyl groups including (C₇₋₂₀ aralkyl)oxycarbonyl groups such as benzyloxycarbonyl group and p-methoxybenzyloxycarbonyl group; and alkylidene groups including aliphatic alkylidene groups such as methylidene, ethylidene, propylidene, isopropylidene, butylidene, isobutylidene, pentylidene, cyclopentylidene, hexylidene, and cyclohexylidene groups, and aromatic alkylidene groups such as benzylidene and methylphenylmethylidene. The amino-protecting groups are not limited to these, and any groups that are conventionally used in the field of organic synthesis can be used.

The protected or unprotected amino groups further include mono-substituted amino groups within ranges not adversely affecting the reactions and the properties of the resulting crosslinked polymers. Examples of the mono-substituted amino groups include alkylamino groups such as methylamino group, ethylamino group, propylamino group, butylamino group, and t-butylamino group; cycloalkylamino groups such as cyclohexylamino group; arylamino groups such as phenylamino group; and aralkylamino groups such as benzylamino group.

Examples of "protecting groups" of the protected or unprotected hydroxyl groups and protected or unprotected mercapto groups in R^{a}, R^{b}, R^{c}, and R^{d} include alkyl groups including alkyl groups having one to six carbon atoms, such as methyl, ethyl, propyl, isopropyl, butyl, t-butyl, pentyl, and hexyl groups; cycloalkyl groups including cycloalkyl groups having three to fifteen members, such as cyclopentyl group and cyclohexyl group; aralkyl groups including aralkyl groups having seven to twenty carbon atoms, such as benzyl group; substituted methyl groups including substituted methyl groups having, in total, about two to about ten carbon atoms, such as methoxymethyl, benzyloxymethyl, t-butoxymethyl, and 2-methoxyethoxymethyl groups; substituted ethyl groups such as 1-ethoxyethyl and 1-methyl-1-methoxyethyl groups; acyl groups including aliphatic acyl groups having one to ten carbon atoms, such as formyl, acetyl, propionyl, butyryl, isobutyryl, valeryl, and pivaloyl groups, alicyclic acyl groups having four to twenty carbon atoms, such as cyclohexylcarbonyl group, and aromatic acyl groups having seven to twenty carbon atoms, such as benzoyl and naphthoyl groups; alkoxycarbonyl groups including (C₁₋₄ alkoxy)-carbonyl groups such as methoxycarbonyl, ethoxycarbonyl, and t-butoxycarbonyl; and aralkyloxycarbonyl groups including (C₇₋₂₀ aralkyl)oxycarbonyl groups such as benzyloxycarbonyl group and p-methoxybenzyloxycarbonyl group. The hydroxyl-protecting groups and mercapto-protecting groups are not limited to these, and protecting groups conventionally used in the field of organic synthesis can be used.

The hydrocarbon groups in R^{a}, R^{b}, R^{c}, and R^{d} include aliphatic hydrocarbon groups, alicyclic hydrocarbon groups, aromatic hydrocarbon groups, and groups comprising these groups combined with each other. Examples of the aliphatic hydrocarbon groups include linear or branched-chain alkyl groups having about one to about twenty carbon atoms, such as methyl, ethyl, propyl, isopropyl, butyl, isobutyl, s-butyl, t-butyl, pentyl, hexyl, decyl, and dodecyl groups, of which those having about one to about ten carbon atoms are preferred, and those having about one to about six carbon atoms are more preferred; linear or branched-chain alkenyl groups having about two to about twenty carbon atoms, such as vinyl, allyl, 1-butenyl, and 3-methyl-4-pentenyl groups, of which those having about two to about ten carbon atoms are preferred, and those having about two to about five carbon atoms are more preferred; and linear or branched-chain alkynyl groups having about two to about twenty carbon atoms, such as ethynyl, propynyl, 1-butynyl, and 2-butynyl groups, of which those having about two to about ten carbon atoms are preferred, and those having about two to about five carbon atoms are more preferred.

Examples of the alicyclic hydrocarbon groups include monocyclic alicyclic hydrocarbon groups including cycloalkyl groups having about three to about twenty members, such as cyclopropyl, cyclobutyl, cyclopentyl, cyclohexyl, and cyclooctyl groups, of which those having about three to about fifteen members are preferred, and those having about three to about twelve members are more preferred, and cycloalkenyl groups having about three to about twenty members, such as cyclopropenyl, cyclobutenyl, cyclopentenyl, and cyclohexenyl groups, of which those having about three to about fifteen members are preferred, and those having about three to about ten members are more preferred; and bridged hydrocarbon groups comprising bridged carbon rings each having about two to about four rings, such as adamantane ring, perhydroindene ring, decalin ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, and norbornene ring. Examples of the aromatic hydrocarbon groups include aromatic hydrocarbon groups having about six to about twenty carbon atoms, such as phenyl and naphthyl groups, of which those having about six to about fourteen carbon atoms are preferred.

Hydrocarbon groups comprising an aliphatic hydrocarbon group and an alicyclic hydrocarbon group combined with each other include cycloalkyl-alkyl groups including (C₃₋₂₀ cycloalkyl)-(C₁₋₄ alkyl) groups, such as cyclopentylmethyl, cyclohexylmethyl, and 2-cyclohexylethyl groups. Hydrocarbon groups comprising an aliphatic hydrocarbon group and an aromatic hydrocarbon group combined with each other include aralkyl groups such as aralkyl groups having seven to eighteen carbon atoms; and alkyl-substituted aryl groups such as phenyl group or naphthyl group substituted with about one to about four (C₁₋₄ alkyl) groups.

The aliphatic hydrocarbon groups, alicyclic hydrocarbon groups, aromatic hydrocarbon groups, and groups comprising these groups combined with each other may each have one or more substituents. Such substituents are not specifically limited, as long as they do not adversely affect the reactions and the physical properties of the crosslinked polymers.

Aromatic rings corresponding to the bivalent aromatic cyclic groups in Y^{a}, Y^{b}, Y^{c}, and Y^{d} include monocyclic or polycyclic aromatic carbon rings and heteroaromatic rings. The term "polycyclic" substance used herein means and includes not only one having a fused-ring structure in which adjacent two rings posses two or more atoms in common, but also one having a structure in which two or more rings are combined with each other with the interposition of one or more linkage groups. Examples of the linkage groups are single bond, bivalent hydrocarbon groups (e.g., methylene group and vinylene group), oxygen atom, nitrogen atom, silicon atom, and sulfur atom.

The monocyclic aromatic hydrocarbon rings include benzene ring. Examples of the polycyclic aromatic hydrocarbon rings include those having a fused-ring structure in which two or more aromatic rings posses two or more atoms in common, such as naphthalene ring, anthracene ring, phenanthrene ring, triphenylene ring, and pyrene ring; and those having a structure in which two or more aromatic rings are combined with each other with the interposition of a linkage group such as single bond, such as biphenyl ring, biphenylene ring, fluorene ring, and stilbene ring. The heteroaromatic rings include monocyclic or polycyclic heteroaromatic rings containing one or more hetero atoms such as oxygen atoms, sulfur atoms, and nitrogen atoms. Specific examples of the heteroaromatic rings include monocyclic rings such as furan ring, thiophene ring, pyridine ring, and picoline ring; and polycyclic rings such as quinoline ring, isoquinoline ring, acridine ring, and phenazine ring. These rings may each have one or more substituents, as long as they do not adversely affect the reactions and the physical properties of the crosslinked polymers.

Nonaromatic rings corresponding to the bivalent nonaromatic cyclic groups in Y^{a}, Y^{b}, Y^{c}, and Y^{d} include monocyclic or polycyclic alicyclic carbon rings and nonaromatic heterocyclic rings. The alicyclic carbon rings include monocyclic alicyclic carbon rings including cycloalkane rings having about three to about twenty members, such as cyclopropane ring, cyclobutane ring, cyclopentane ring, cyclohexane ring, and cyclooctane ring, of which those having about three to about fifteen members are preferred, and those having about three to about twelve members are more preferred, and cycloalkene rings having about three to about twenty members, such as cyclopropene ring, cyclobutene ring, cyclopentene ring, and cyclohexene ring, of which those having about three to about fifteen members are preferred, and those having about three to about ten members are more preferred; and bridged carbon rings comprising about two to about six rings, such as adamantane ring, perhydroindene ring, decalin ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, and norbornene ring. Examples of the nonaromatic heterocyclic rings include five-or six-membered heterocyclic rings having at least one hetero atom selected from oxygen atoms, sulfur atoms, and nitrogen atoms, and fused rings containing these. The nonaromatic cyclic groups may each have one or more substituents, as long as they do not adversely affect the reactions and the physical properties of the crosslinked polymers.

Representative examples of the compounds represented by Formula (1) include adamantane derivatives having an adamantane ring in the central skeleton, such as 1,3,5,7-adamantanetetracarboxylic acid, 1,3,5,7-tetrakis(4-carboxyphenyl)adamantane, and 1,3,5,7-adamantanetetramine; norbornane or norbornene derivatives having a norbornane ring or norbornene ring in the central skeleton, such as 2,3,5,6-norbornanetetracarboxylic acid, 2,3,5,6-norbornenetetracarboxylic acid, 2,3,5,6-tetrakis(hydroxymethyl)norbornane, and 2,3,5,6-tetrakis(hydroxymethyl)norbornene; tetraarylmethane derivatives having a carbon atom in the central skeleton, such as tetrakis(4-carboxyphenyl)methane; butane derivatives having a butane skeleton as the central skeleton, such as erythritol; and porphyrin derivatives having, as the central skeleton, a porphyrin ring containing functional groups in four nitrogen-containing rings of porphyrin.

The compounds represented by Formula (1) preferably have a three-dimensionally bulky structure, i.e., a structure having a large volume. They more preferably have a substantially tetrahedral structure with Z^{a} as the center and R^{a}, R^{b}, R^{c}, and R^{d} as vertices.

Representative examples of the compounds B in the prepolymers according to the present invention include the compounds represented by Formula (2). In Formula (2), Ring Z^{b} represents a monocyclic or polycyclic aromatic or nonaromatic ring; and R^{e}, R^{f}, R^{g}, and R^{h} are substituents bound to Ring Z^{b}, are the same as or different from one another, and each represent a protected or unprotected amino group, a protected or unprotected hydroxyl group, a protected or unprotected mercapto group, or a protected or unprotected carboxyl group. In these compounds, R^{e}, R^{f}, R^{g}, and R^{h} correspond to the functional groups or sets of functional groups.

Of R^{e}, R^{f}, R^{g}, and R^{h}, a pair of groups (for example, R^{e} and R^{f}, or R^{g} and R^{h}) is preferably bound at the 1- and 2-positions (alpha-positions) or 1- and 3-positions (beta-positions), respectively, of the constitutional atoms of Ring Z^{b}, for the sake of easy cyclization. The pair of groups is preferably one selected from a combination of two amino groups; a combination of an amino group and a hydroxyl group; a combination of an amino group and a mercapto group; and a combination of two carboxyl groups. Compounds B of this type can form a ring with a compound A having one carboxyl group or two amino groups as the functional groups or sets of functional groups.

The monocyclic or polycyclic aromatic ring in Ring Z^{b} includes aromatic carbon rings and heteroaromatic rings. The monocyclic aromatic carbon rings include benzene ring. Examples of the polycyclic aromatic carbon rings include rings having a fused-ring structure in which two or more aromatic rings each posses two or more atoms in common, such as naphthalene ring, anthracene ring, phenanthrene ring, triphenylene ring, and pyrene ring; and rings having a structure in which two or more aromatic rings are combined with each other with the interposition of one or more linkage groups, such as biphenyl ring, biphenylene ring, fluorene ring, and stilbene ring. Examples of the linkage groups include single bond, bivalent hydrocarbon group such as methylene group and vinylene group, oxygen atom, nitrogen atom, silicon atom, and sulfur atom. The heteroaromatic rings include monocyclic or polycyclic heteroaromatic rings containing one or more hetero atoms such as oxygen atom, sulfur atom, and nitrogen atom. Specific examples of the heteroaromatic rings include monocyclic rings such as furan ring, thiophene ring, pyridine ring, and picoline ring; and polycyclic rings such as quinoline ring, isoquinoline ring, acridine ring, and phenazine ring.

The monocyclic or polycyclic nonaromatic ring in Ring Z^{b} includes alicyclic hydrocarbon rings and nonaromatic heterocyclic rings. The nonaromatic rings include nonaromatic rings corresponding to the nonaromatic cyclic groups in Z^{a}. The monocyclic or polycyclic aromatic or nonaromatic rings in Ring Z^{b} may each have one or more substituents within ranges not adversely affecting the reactions and the physical properties of the crosslinked polymers.

The "protecting groups" of the protected or unprotected amino groups in R^{e}, R^{f}, R^{g}, and R^{h} include protecting groups similar to the amino-protecting groups in R^{a}, R^{b}, R^{c}, and R^{d}. As the amino-protecting groups, protecting groups that can protect plural amino groups simultaneously (polyfunctional protecting groups) can also be used. Such polyfunctional protecting groups include, for example, carbonyl group, oxalyl group, and butane-2,3-diylidene group. By using a protecting group of this type, two of R^{e}, R^{f}, R^{g}, and R^{h} are protected by one polyfunctional protecting group simultaneously to thereby form a ring adjacent to Ring Z^{b}. The protected or unprotected amino groups in R^{e}, R^{f}, R^{g}, and R^{h} further include mono-substituted amino groups within ranges not adversely affecting the reactions and the physical properties of the resulting crosslinked polymers. Examples of the mono-substituted amino groups include alkylamino groups such as methylamino group, ethylamino group, propylamino group, butylamino group, and t-butylamino group; cycloalkylamino groups such as cyclohexylamino group; arylamino groups such as phenylamino group; and aralkylamino groups such as benzylamino group.

The "protecting groups" for the protected or unprotected hydroxyl groups, protected or unprotected mercapto groups, and protected or unprotected carboxyl groups in R^{e}, R^{f}, R^{g}, and R^{h} include those exemplified as the protecting groups for hydroxyl group, mercapto group, and carboxyl group in R^{a}, R^{b}, R^{c}, and R^{d}.

Representative examples of the compounds represented by Formula (2) include after-mentioned compounds represented by Formula (6), as well as compounds in which R^{e}, R^{f}, R^{g}, and R^{h} are each a protected or unprotected carboxyl group, such as 1,2,4,5-benzenetetracarboxylic acid or derivatives thereof.

The prepolymers according to the present invention also include a prepolymer as a relation product between the at least one adamantanepolycarboxylic acid derivative represented by Formula (3), (4), or (5) and the polyamine derivative represented by Formula (6), wherein the prepolymer comprises an amide bond, an ester bond, or a thioester bond formed as a result of a reaction of the protected or unprotected carboxyl group or haloformyl group in R¹, R², R³, and R⁴ of the adamantanepolycarboxylic acid derivative represented by Formula (3), (4), or (5) with the protected or unprotected amino group, protected or unprotected hydroxyl group, or protected or unprotected mercapto group in R⁵, R⁶, R⁷, and R⁸ of the polyamine derivative represented by Formula (6). In the present description, a compound in which the protected or unprotected carboxyl group is not directly bound to the adamantane ring is also referred to as an "adamantanepolycarboxylic acid derivative" for the sake of convenience.

The protected or unprotected carboxyl groups and the haloformyl groups in R¹, R², R³, and R⁴ of Formulae (3), (4), and (5) are as with the protected or unprotected carboxyl groups and the haloformyl groups typically in R^{a}. The bivalent aromatic or nonaromatic cyclic groups in Y¹, Y², Y³, and Y⁴ are as with the bivalent aromatic or nonaromatic cyclic groups in Y^{a}. The protected or unprotected carboxyl groups and the hydrocarbon groups in L¹ and L³ are as with the protected or unprotected carboxyl groups and the hydrocarbon groups typically in R^{a}.

The compounds represented by Formulae (3), (4), and (5) are adamantanepolycarboxylic esters when R¹, R², R³, and R⁴ are each, for example, an alkoxycarbonyl group or an aryloxycarbonyl group; they are adamantanepolycarboxylic acid amides when R¹, R², R³, and R⁹ are each a substituted or unsubstituted carbamoyl group; and they are adamantanepolycarbonyl halides when R¹, R², R³, and R⁴ are each a haloformyl group.

Preferred examples of R¹, R², R³, and R⁴ include carboxyl group, (C₁₋₆ alkoxy) -carbonyl groups, (C₁₋₄ alkoxy)₁₋₂-(C₁₋₄ alkoxy)-carbonyl groups, N-substituted carbamoyl groups, tetrahydropyranyloxycarbonyl group, tetrahydrofuranyloxycarbonyl group, aryloxycarbonyl groups, trialkylsilyloxycarbonyl groups, and haloformyl groups.

The adamantanepolycarboxylic acid derivatives represented by Formula (3) (adamantanetetracarboxylic acids and derivatives thereof) include compounds wherein all the four functional groups, R¹, R², R³, and R⁴, are carboxyl groups; compounds wherein one of the four functional groups is a protected carboxyl group or a haloformyl group; compounds wherein two of the four functional groups are each a protected carboxyl group or a haloformyl group; compounds wherein three of the four functional groups are each a protected carboxyl group or a haloformyl group; and compounds wherein all the four functional groups are each a protected carboxyl group or a haloformyl group.

Representative examples of the adamantanepolycarboxylic acid derivatives represented by Formula (3) include 1,3,5,7-adamantanetetracarboxylic acid, 1,3,5,7-tetrakis(4-carboxyphenyl)adamantane; 1-methoxycarbonyl-3,5,7-adamantanetricarboxylic acid, 1-(t-butoxycarbonyl)-3,5,7-adamantanetricarboxylic acid, 1-tetrahydropyranyl (THP)oxycarbonyl-3,5,7-adamantanetricarboxylic acid, 1-phenoxycarbonyl-3,5,7-adamantanetricarboxylic acid, 1-methoxymethyl (MEM)oxycarbonyl-3,5,7-adamantanetricarboxylic acid, 1-trimethylsilyl (TMS)oxycarbonyl-3,5,7-adamantanetricarboxylic acid, 1,3,5-tricarboxy-7-adamantanecarbonyl chloride, 1-diethylcarbamoyl-3,5,7-adamantanetricarboxylic acid, 1-(1-pyrrolidinylcarbonyl)-3,5,7-adamantanetricarboxylic acid, 1,3,5-tris(4-carboxyphenyl)-7-(4-methoxycarbonylphenyl)adamantane; 1,3-bis(methoxycarbonyl)-5,7-adamantanedicarboxylic acid, 1,3-bis(t-butoxycarbonyl)-5,7-adamantanedicarboxylic acid, 1,3-bis(tetrahydropyranyloxycarbonyl)-5,7-adamantanedicarboxylic acid, 1,3-bis(phenoxycarbonyl)-5,7-adamantanedicarboxylic acid, 1,3-bis(methoxymethyloxycarbonyl)-5,7-adamantanedicarboxylic acid, 1,3-bis(trimethylsilyloxycarbonyl)-5,7-adamantanedicarboxylic acid, 1,3-dicarboxy-5,7-adamantanedicarbonyl dichloride, 1,3-bis(diethylcarbamoyl)-5,7-adamantanedicarboxylic acid, 1,3-bis(1-pyrrolidinylcarbonyl)-5,7-adamantanedicarboxylic acid, 1,3-bis(4-carboxyphenyl)-5,7-bis(4-methoxycarbonylphenyl)adamantane; 1, 3, 5-tris(methoxycarbonyl)-7-adamantanemonocarboxylic acid, 1,3,5-tris(t-butoxycarbonyl)-7-adamantanemonocarboxylic acid, 1,3,5-tris(tetrahydropyranyloxycarbonyl)-7-adamantanemonocarboxylic acid, 1,3,5-tris(phenoxycarbonyl)-7-adamantanemonocarboxylic acid, 1,3,5-tris(methoxymethyloxycarbonyl)-7-adamantanemonocarboxylic acid, 1,3,5-tris(trimethylsilyloxycarbonyl)-7-adamantanemonocarboxylic acid, 1-carboxy-3,5,7-adamantanetricarbonyl trichloride, 1,3,5-tris(diethylcarbamoyl)-7-adamantanemonocarboxylic acid, 1,3,5-tris(1-pyrrolidinylcarbonyl)-7-adamantanemonocarboxylic acid, 1-(4-carboxyphenyl)-3,5,7-tris(4-methoxycarbonylphenyl)adamantane; 1,3,5,7-tetrakis(methoxycarbonyl)adamantane, 1,3,5,7-tetrakis(t-butoxycarbonyl)adamantane, 1,3,5,7-tetrakis(tetrahydropyranyloxycarbonyl)adamantane, 1,3,5,7-tetrakis(phenoxycarbonyl)adamantane, 1,3,5,7-tetrakis(methoxymethyloxycarbonyl)adamantane, 1,3,5,7-tetrakis(trimethylsilyloxycarbonyl)adamantane, 1,3,5,7-adamantanetetracarbonyl tetrachloride, 1,3,5,7-tetrakis(diethylcarbamoyl)adamantane, 1,3,5,7-tetrakis(1-pyrrolidinylcarbonyl)adamantane, and 1,3,5,7-tetrakis(4-methoxycarbonylphenyl)adamantane.

Each of these adamantanetetracarboxylic acids and derivatives thereof can be used alone or in combination.

The adamantanepolycarboxylic acid derivatives represented by Formula (4) (adamantanetricarboxylic acids and derivatives thereof) include compounds in which all the three functional groups are carboxyl groups; compounds in which one of the three functional groups is a protected carboxyl group or a haloformyl group; compounds in which two of the three functional groups are each a protected carboxyl group or a haloformyl group; and compounds in which all the three functional groups are each a protected carboxyl group or a haloformyl group.

Representative examples of the adamantanepolycarboxylic acid derivatives represented by Formula (4) include 1,3,5-adamantanetricarboxylic acid, 7-methyl-1,3,5-adamantanetricarboxylic acid, 7-phenyl-1,3,5-adamantanetricarboxylic acid, 1,3,5-tris(4-carboxyphenyl)adamantane, 1,3,5-tris(4-carboxyphenyl)-7-methyladamantane, 1,3,5-tris(4-carboxyphenyl)-7-phenyladamantane; 1-methoxycarbonyl-3,5-adamantanedicarboxylic acid, 1-(t-butoxycarbonyl)-3,5-adamantanedicarboxylic acid, 1-tetrahydropyranyloxycarbonyl-3,5-adamantanedicarboxylic acid, 1-phenoxycarbonyl-3,5-adamantanedicarboxylic acid, 1-methoxymethyloxycarbonyl-3,5-adamantanedicarboxylic acid, 1-trimethylsilyloxycarbonyl-3,5-adamantanedicarboxylic acid, 1,3-dicarboxy-5-adamantanecarbonyl chloride, 1-diethylcarbamoyl-3,5-adamantanedicarboxylic acid, 1-pyrrolidinylcarbonyl-3,5-adamantanedicarboxylic acid, 1,3-bis(4-carboxyphenyl)-5-(4-methoxycarbonylphenyl)adamantane; 1,3-bis(methoxycarbonyl)-5-adamantanemonocarboxylic acid, 1,3-bis(t-butoxycarbonyl)-5-adamantanemonocarboxylic acid, 1,3-bis(tetrahydropyranyloxycarbonyl)-5-adamantanemonocarboxylic acid, 1,3-bis(phenoxycarbonyl)-5-adamantanemonocarboxylic acid, 1,3-bis(methoxymethyloxycarbonyl)-5-adamantanemonocarboxylic acid, 1,3-bis(trimethylsilyloxycarbonyl)-5-adamantanemonocarboxylic acid, 1-carboxy-3,5-adamantanedicarbonyl dichloride, 1,3-bis(diethylcarbamoyl)-5-adamantanemonocarboxylic acid, 1,3-bis(1-pyrrolidinylcarbonyl)-5-adamantanemonocarboxylic acid, 1-(4-carboxyphenyl)-3,5-bis(4-methoxycarbonylphenyl)-adamantane; 1,3, 5-tris (methoxycarbonyl) adamantane, 1,3,5-tris(t-butoxycarbonyl)adamantane, 1,3,5-tris(tetrahydropyranyloxycarbonyl)adamantane, 1,3,5-tris(phenoxycarbonyl)adamantane, 1,3,5-tris(methoxymethyloxycarbonyl)adamantane, 1,3,5-tris(trimethylsilyloxycarbonyl)adamantane, 1,3,5-adamantanetricarbonyl trichloride, 1,3,5-tris(diethylcarbamoyl)adamantane, 1,3,5-tris (1-pyrrolidinylcarbonyl)adamantane, and 1,3,5-tris(4-methoxycarbonylphenyl)adamantane.

Each of these adamantanetricarboxylic acids and derivatives thereof can be used alone or in combination.

The adamantanepolycarboxylic acid derivatives represented by Formula (5) (adamantanedicarboxylic acids and derivatives thereof) include compounds in which all the two functional groups are carboxyl groups; compounds in which one of the two functional groups is a protected carboxyl group or a haloformyl group; and compounds in which all the two functional groups are each a protected carboxyl group or a haloformyl group.

Representative examples of the adamantanepolycarboxylic acid derivatives represented by Formula (5) include 1,3-adamantanedicarboxylic acid, 5-methyl-1,3-adamantanedicarboxylic acid, 5-phenyl-1,3-adamantanedicarboxylic acid, 1,3-bis(4-carboxyphenyl)adamantane, 1,3-bis(4-carboxyphenyl)-5-methyladamantane, 1,3-bis(4-carboxyphenyl)-5-phenyladamantane; 1-methoxycarbonyl-3-adamantanemonocarboxylic acid, 1-(t-butoxycarbonyl)-3-adamantanemonocarboxylic acid, 1-tetrahydropyranyloxycarbonyl-3-adamantanemonocarboxylic acid, 1-phenoxycarbonyl-3-adamantanemonocarboxylic acid, 1-methoxymethyloxycarbonyl-3-adamantanemonocarboxylic acid, 1-trimethylsilyloxycarbonyl-3-adamantanemonocarboxylic acid, 1-carboxy-3-adamantanecarbonyl chloride, 1-diethylcarbamoyl-3-adamantanemonocarboxylic acid, 1-pyrrolidinylcarbonyl-3-adamantanemonocarboxylic acid, 1-(4-carboxyphenyl)-3-(4-methoxycarbonylphenyl)adamantane; 1,3-bis(methoxycarbonyl)adamantane, 1,3-bis(t-butoxycarbonyl)adamantane, 1,3-bis(tetrahydropyranyloxycarbonyl)adamantane, 1,3-bis(phenoxycarbonyl)adamantane, 1,3-bis(methoxymethyloxycarbonyl)adamantane, 1,3-bis(trimethylsilyloxycarbonyl)adamantane, 1,3-adamantanedicarbonyl dichloride, 1,3-bis(diethylcarbamoyl)adamantane, 1,3-bis(1-pyrrolidinylcarbonyl)adamantane, and 1,3-bis(4-methoxycarbonylphenyl)adamantane.

Each of these adamantanedicarboxylic acids and derivatives thereof can be used alone or in combination.

The adamantanepolycarboxylic acid derivatives represented by Formulae (3), (4), and (5) can be obtained, for example, by a known method or by using a known organic synthesis reaction or a known technique for introducing a protecting group into carboxyl group, such as esterification or amidation.

The monocyclic or polycyclic aromatic or nonaromatic ring in Ring Z¹ of Formula (6) is as with the monocyclic or polycyclic aromatic or nonaromatic ring in Ring Z^{b}. The protected or unprotected amino groups, protected or unprotected hydroxyl groups, and protected or unprotected mercapto groups in R⁵, R⁶, R⁷, and R⁸ are as with the protected or unprotected amino groups, protected or unprotected hydroxyl groups, and protected or unprotected mercapto groups typically in R^{e}. At least two of R⁵, R⁶, R⁷, and R⁸ in Formula (6) are each a protected or unprotected amino group. According to the present invention, when at least R⁶ and R⁷, for example, are each a protected or unprotected amino group, R⁵ with R⁶, and R⁷ with R⁸ are each preferably at such positions that they can react with any of R¹, R², and R³ of the adamantanepolycarboxylic acid represented by Formula (3), (4), or (5) to thereby form a ring. These groups are more preferably bound at the 1- and 2-positions (alpha-positions) or the 1- and 3-positions (beta-positions) of constitutional atoms of Ring Z¹ so as to form a five-membered ring or six-membered ring.

The polyamine derivatives represented by Formula (6) include (i) compounds in which all the protected or unprotected amino groups of R⁵, R⁶, R⁷, and R⁸ are unprotected amino groups; (ii) compounds in which at least one of R⁵, R⁶, R⁷, and R⁸ is an amino group protected by an alkylidene group (i.e., imine derivatives); (iii) compounds in which at least one of R⁵, R⁶, R⁷, and R⁸ is an acylamino group (i.e., amide derivatives); (iv) compounds in which at least one of R⁵, R⁶, R⁷, and R⁸ is an amino group protected by an alkoxycarbonyl group or aralkyloxycarbonyl group (i.e., carbamic ester derivatives); and (v) compounds in which at least one of R⁵, R⁶, R⁷, and R⁸ is a mono-substituted amino group.

Representative compounds of the polyamine derivatives as exemplified as the compounds (i) to (v) are shown below, by taking compounds in which Ring Z¹ is a benzene ring, and the number of protecting groups, if any, is four or two as an example. However, the compounds are not limited to these. For example, when Ring Z¹ is a biphenyl ring, compounds corresponding to the following compounds can be listed.

Representative examples of the compounds (i) in which all the protected or unprotected amino groups of R⁵, R⁶, R⁷, and R⁸ are unprotected amino groups include 1,2,4,5-tetraaminobenzene, 1,4-diamino-2,5-dihydroxybenzene, 1,5-diamino-2,4-dihydroxybenzene, 1,4-diamino-2,5-dimercaptobenzene, and 1,5-diamino-2,4-dimercaptobenzene.

Representative examples of the compounds (ii) in which at least one of R⁵, R⁶, R⁷, and R⁸ is an amino group protected by an alkylidene group (i.e., imine derivatives) include compounds represented by the following formulae:

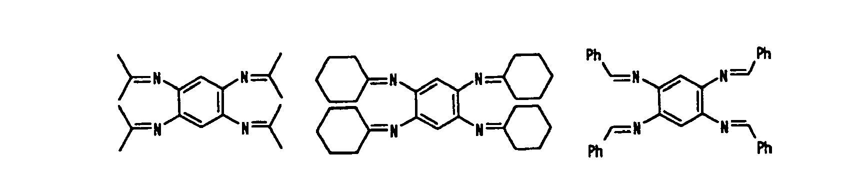

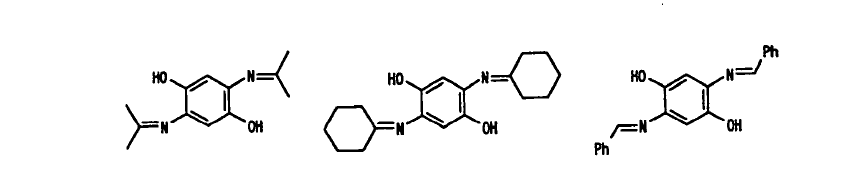

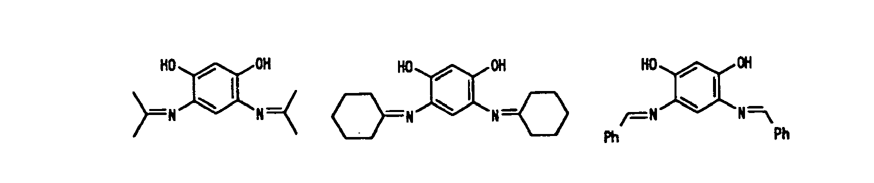

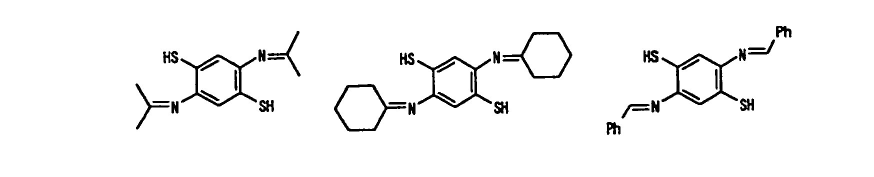

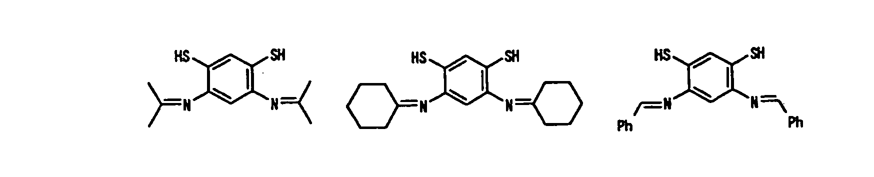

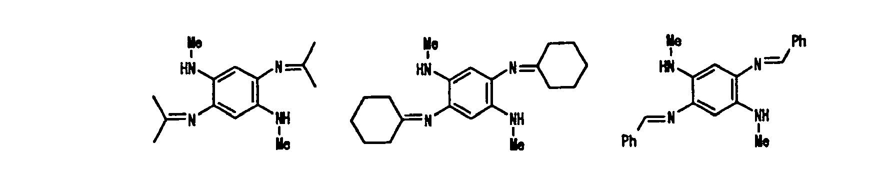

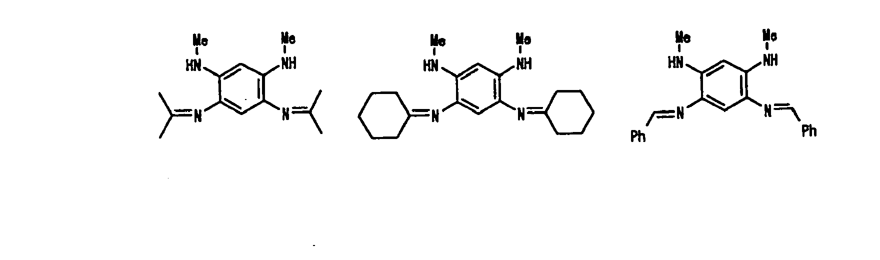

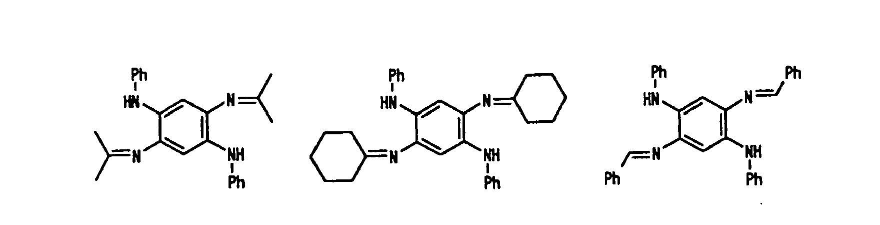

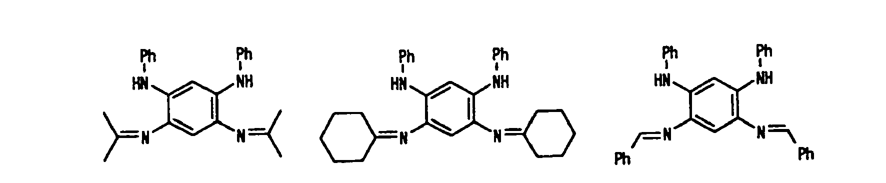

The imine derivatives include imine derivatives of Formula (6) in which R⁵ and R⁶ are each an amino group protected by an alkylidene group, including those in which R⁷ and R⁸ are amino groups, such as N,N" -diisopropylidene-1,2,4,5-benzenetetramine, N,N"'-diisopropylidene-1,2,4,5-benzenetetramine, N,N" -dicyclohexylidene-1,2,4,5-benzenetetramine, N,N"'-dicyclohexylidene-1,2,4,5-benzenetetramine, N,N''-dibenzylidene-1,2,4,5-benzenetetramine, and N,N"'-dibenzylidene-1,2,4,5-benzenetetramine; and those in which R⁷, R⁸ are each an amino group protected by an alkylidene group, such as N,N',N",N"'-tetraisopropylidene-1,2,4,5-benzenetetramine, N,N',N'',N'''-tetracyclohexylidene-1,2,4,5-benzenetetramine, and N,N',N'',N'''-tetrabenzylidene-1,2,4,5-benzenetetramine.

The imine derivatives also include imine derivatives of Formula (6) in which R⁵ and R⁶ are each an amino group protected by an alkylidene group, including those in which R⁷ and R⁸ are each a mono-substituted amino group, such as N,N"-diisopropylidene-N',N"'-dimethyl-1,2,4,5-benzenetetramine, N,N'''-diisopropylidene-N',N''-dimethyl-1,2,4,5-benzenetetramine, N,N''-dicyclohexylidene-N',N'''-dimethyl-1,2,4,5-benzenetetramine, N,N'''-dicyclohexylidene-N',N"'-dimethyl-1,2,4,5-benzenetetramine, N,N''-dibenzylidene-N',N"'-dimethyl-1,2,4,5-benzenetetramine, N,N'''-dibenzylidene-N',N'''-dimethyl-1,2,4,5-benzenetetramine, N,N''-diisopropylidene-N',N'''-diphenyl-1,2,4,5-benzenetetramine, N,N'''-diisopropylidene-N',N''-diphenyl-1,2,4,5-benzenetetramine, N,N''-dicyclohexylidene-N',N'''-diphenyl-1,2,4,5-benzenetetramine, N,N"'-dicyclohexylidene-N',N'''-diphenyl-1,2,4,5-benzenetetramine, N,N''-dibenzylidene-N',N'''-diphenyl-1,2,4,5-benzenetetramine, and N,N'''-dibenzylidene-N',N'''-diphenyl-1,2,4,5-benzenetetramine.

In addition to the above compounds, the imine derivatives further include imine derivatives in which R⁷ and R⁸ are each a hydroxyl group, such as N,N'-diisopropylidene-2,5-dihydroxy-1,4-benzenediamine, N,N'-diisopropylidene-2,4-dihydroxy-1,5-benzenediamine, N,N'-dicyclohexylidene-2,5-dihydroxy-1,4-benzenediamine, N,N'-dicyclohexylidene-2,4-dihydroxy-1,5-benzenediamine, N,N'-dibenzylidene-2,5-dihydroxy-1,4-benzenediamine, and N,N'-dibenzylidene-2,4-dihydroxy-1,5-benzenediamine; and imine derivatives in which R⁷ and R⁸ are each a mercapto group, such as N,N'-diisopropylidene-2,5-dimercapto-1,4-benzenediamine, N,N'-diisopropylidene-2,4-dimercapto-1,5-benzenediamine, N,N'-dicyclohexylidene-2,5-dimercapto-1,4-benzenediamine, N,N'-dicyclohexylidene-2,4-dimercapto-1,5-benzenediamine, N,N'-dibenzylidene-2,5-dimercapto-1,4-benzenediamine, and N,N'-dibenzylidene-2,4-dimercapto-1,5-benzenediamine.

The compounds (iii) in which at least one of R⁵, R⁶, R⁷ and R⁸ is an acylamino group (i.e., amide derivatives) include, for example, compounds represented by the following formulae.

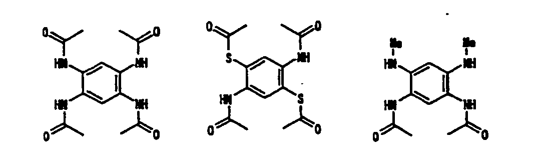

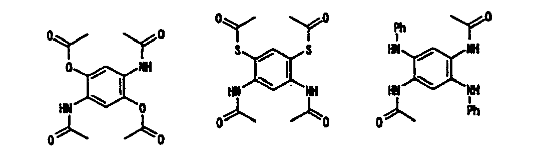

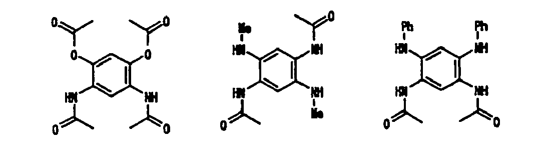

The amide derivatives includes amide derivatives of Formula (6) in which R⁵ and R⁶ are each an acylamino group, including those in which R⁷ and R⁸ are each an acylamino group, such as 1,2,4,5-tetrakis (acetamino) benzene; those in which R⁷ and R⁸ are each a mono-substituted amino group, such as 1,4-bis(acetamino)-2,5-bis(methylamino)benzene, 1,5-bis (acetamino)-2,4-bis (methylamino) benzene, 1,4-bis(methoxycarbonylamino)-2,5-bis(methylamino)benzene, 1,5-bis(methoxycarbonylamino)-2,4-bis(methylamino)benzene, 1,4-bis (acetamino)-2,5-bis (phenylamino) benzene, and 1,5-bis (acetamino) -2,4-bis (phenylamino)benzene; those in which R⁷ and R⁸ are each a protected hydroxyl group, such as 1,4-bis (acetamino)-2,5-diacetoxybenzene, 1,5-bis(acetamino) -2,4-diacetoxybenzene, 1,4-bis(methoxycarbonylamino)-2,5-dimethoxycarbonyloxybenzene, and 1,5-bis(methoxycarbonylamino)-2,4-dimethoxycarbonyloxybenzene; and those in which R⁷ and R⁸ are each a protected mercapto group, such as 1,4-bis (acetamino)-2,5-diacetylthiobenzene and 1,5-bis (acetamino)-2,4-diacetylthiobenzene.

The compounds (iv) in which at least one of R⁵, R⁶, R⁷ and R⁸ is an amino group protected by an alkoxycarbonyl group or an aralkyloxycarbonyl group (i.e., carbamic ester derivatives) include, for example, compounds represented by following formulae:

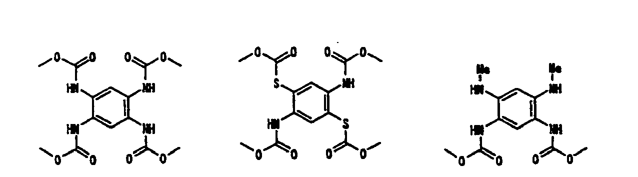

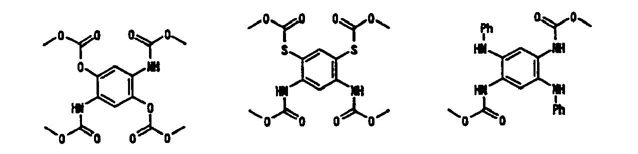

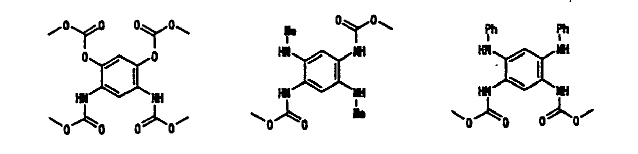

The carbamic ester derivatives include carbamic ester derivatives of Formula (2) in which R⁵ and R⁶ are each an alkoxycarbonyl group, including those in which R⁷ and R⁸ are each an alkoxycarbonyl group, such as 1,2,4,5-tetrakis(acetylamino)benzene; those in which R⁷ and R⁸ are each a mono-substituted amino group, such as 1,4-bis(methoxycarbonylamino)-2,5-bis(phenylamino)benzene, 1,5-bis(methoxycarbonylamino)-2,4-bis(phenylamino)benzene, 1,2,4,5-tetrakis(methylamino)benzene, and 1,2,4,5-tetrakis(phenylamino)benzene; those in which R⁷ and R⁸ are each a protected hydroxyl group, such as 1,4-bis(methoxycarbonylamino)-2,5-dimethoxycarbonyloxybenzene, and 1,5-bis(methoxycarbonylamino)-2,4-dimethoxycarbonyloxybenzene; and those in which R⁷ and R⁸ are each a protected mercapto group, such as 1,4-bis(methoxycarbonylamino)-2,5-dimethoxycarbonylthiobenzene, and 1,5-bis(methoxycarbonylamino)-2,4-dimethoxycarbonylthiobenzene.

Representative examples of the compounds (v) in which at least one of R⁵, R⁶, R⁷ and R⁸ is a mono-substituted amino group include compounds of Formula (6) in which R⁵, R⁶ are each a mono-substituted amino group; and R⁷ and R⁸ are each an amino group, such as 1,4-diamino-2,5-bis(methylamino)benzene, 1,5-diamino-2,4-bis(methylamino)benzene, 1,4-diamino-2,5-bis(phenylamino)benzene, and 1,5-diamino-2,4-bis(phenylamino)benzene.

The polyamine derivatives represented by Formula (6) further include, in addition to the above compounds, compounds in which at least two of R⁵ to R⁸ are combined to form a ring together with adjacent atom. The polyamine derivatives of this type include, for example, compounds in which amino groups in the molecule are protected by the protecting group that can protect plural amino groups simultaneously (polyfunctional protecting group). Representative examples of such compounds include a compound corresponding to 1,2,4,5-tetraaminobenzene, except for being protected by two oxalyl groups [a compound of Formula (6) in which Ring Z¹ is a benzene ring; R⁵ and R⁶ are amino groups protected by an oxalyl group; and R⁷ and R⁸ are amino groups protected by an oxalyl group], and a compound corresponding to 1,2,4,5-tetraaminobenzene, except for being protected by two butane-2,3-diylidene groups [a compound of Formula (6) in which Ring Z is a benzene ring; R⁵ and R⁶ are amino groups protected by a butane-2,3-diylidene group; and R⁷ and R⁸ are amino groups protected by a butane-2,3-diylidene group].

Each of these polyamine derivatives can be used alone or in combination.

The polyamine derivative represented by Formula (6) can be prepared by a known method or by using a known organic synthesis reaction or a known technique for introducing a protecting group into an amino group using, for example, acylation, imination, or alkylation.

The reaction between the at least one adamantanepolycarboxylic acid derivative represented by Formula (3), (4), or (5) and the polyamine derivative represented by Formula (6) can be conducted according typically to a regular amidation, esterification or thioesterification reaction.

The reaction is generally carried out in a solvent. Any solvent will do, as long as it can dissolve raw materials and does not adversely affect the reactions. Examples of the solvent are amides such as N,N-dimethylformamide, N,N-dimethylacetamide, and N-methyl-2-pyrrolidone; sulfoxides such as dimethyl sulfoxide; sulfones; nitriles such as acetonitrile, propionitrile, and benzonitrile; ketones such as acetone, methyl ethyl ketone, diethyl ketone, methyl isobutyl ketone, cyclopentanone, and cyclohexanone; esters such as formic esters, acetic esters, propionic esters, benzoic esters, γ-butyrolactone, and propylene glycol monomethyl ether acetate (PGMEA); ethers such as dioxane, tetrahydrofuran, diethyl ether, and ethylene glycol diethyl ether; halogenated hydrocarbons such as dichloromethane, dichloroethane, chloroform, carbon tetrachloride, and chlorobenzene; aromatic hydrocarbons such as benzene, toluene, xylenes, ethylbenzene, and mesitylene; alicyclic hydrocarbons such as cyclohexane and methylcyclohexane; and aliphatic hydrocarbons such as hexane, heptane, and octane. Each of these solvents can be used alone or in combination.

The reaction temperature can be generally selected within the range of -60°C to 200°C, while it varies depending typically on the type of the adamantanepolycarboxylic acid derivative represented by Formula (3), (4), or (5) and the type of the polyamine derivative represented by Formula (6). For example, when the adamantanepolycarboxylic acid derivative is a carbonyl halide and the polyamine derivative is an unprotected amine, the reaction temperature is about - 10°C to about 50°C.

The proportions of the adamantanepolycarboxylic acid derivative and the polyamine derivative can be set within a broad range. The two components can be used in an equivalent amount or one of them can be used in excess. Generally, the polyamine derivative is frequently used in excess. For example, the amount of the polyamine derivative is generally about 0.01 to about 100 equivalents, preferably about 1 to about 50 equivalents, and more preferably about 4 to about 20 equivalents, to the adamantanepolycarboxylic acid.

The reaction can be carried out in the presence of, for example, an appropriate catalyst (e.g., a base catalyst or an acid catalyst), a reactant, and/or a trapping agent (e.g., a base or a dehydrating agent), for accelerating the reaction and/or eliminating the protecting group(s), according to the types of the raw material adamantanepolycarboxylic acid derivative and polyamine derivative.

Thus, the protected or unprotected carboxyl groups or haloformyl groups in R¹, R², R³ and R⁴ of the adamantanepolycarboxylic acid derivative react with the protected or unprotected amino groups, protected or unprotected hydroxyl groups, or protected or unprotected mercapto groups in R⁵, R⁶, R⁷ and R⁸ of the polyamine derivative to form amide bonds, ester bonds, and/or thioester bonds to thereby yield a prepolymer. When one or two of R⁵ to R⁸ are each a protected or unprotected hydroxyl group, or a protected or unprotected mercapto group, amide bonds alone may be formed without the formation of ester bonds and thioester bonds.

The weight-average molecular weight of the resulting prepolymer is, for example, about 200 to about 100000, preferably about 300 to about 50000, and more preferably about 1000 to about 30000. The molecular weight of the prepolymer can be controlled by appropriately adjusting the proportions of the adamantanepolycarboxylic acid derivative and the polyamine derivative and reaction conditions such as reaction temperature and reaction time.

The resulting prepolymer can be isolated by a separation/purification procedure such as precipitation, reprecipitation, crystallization, recrystallization, filtration, extraction, or drying.

The prepolymers include (i) the compound represented by Formula (7) as a reaction product between the adamantanepolycarboxylic acid derivative represented by Formula (3) and the polyamine derivative represented by Formula (6); (ii) the compound represented by Formula (10) as a reaction product between the adamantanepolycarboxylic acid derivative represented by Formula (4) and the polyamine derivative represented by Formula (6); and (iii) the compound represented by Formula (15), as a reaction product between the adamantanepolycarboxylic acid derivative represented by Formula (5) and the polyamine derivative represented by Formula (6). The prepolymers also include mixtures of these prepolymers, and prepolymers each comprising two or more of: a constitutional unit corresponding to the adamantanepolycarboxylic acid derivative represented by Formula (3), a constitutional unit corresponding to the adamantanepolycarboxylic acid derivative represented by Formula (4), and a constitutional unit corresponding to the adamantanepolycarboxylic acid derivative represented by Formula (5).

In Formula (7), Y¹, Y², Y³, and Y⁴ are as defined above; and W¹ represents one of R¹, R², R³, R⁴ and a group represented by Formula (8). In Formula (8), Ring Z¹ is as defined above; and the symbol "R⁵⁻⁸" means any one of R⁵, R⁶, R⁷, and R⁸; X is a bond formed as a result of a reaction between one of R¹ to R⁴ and one of R⁵ to R⁸ and represents an amide bond, an ester bond, or a thioester bond; and A¹ represents one of R⁵, R⁶, R⁷, R⁸, and a group represented by Formula (9). In Formula (9), W¹ and X are as defined above; and the symbol "Y¹⁻⁴" means any one of Y¹, Y², Y³, and Y⁴. At lest one of four W¹s in Formula (7) is the group represented by Formula (8). A¹s, W¹s, and Xs, if present plural in number, may be the same as or different from each other, respectively.

In Formula (10), L¹, Y¹ Y², Y³, and Y⁴ are as defined above; and W² represents one of R², R³, R⁴, and a group represented by Formula (11). In Formula (11), Ring Z¹ is as defined above; the symbol "R⁵⁻⁸" represents any one of R⁵, R⁶, R⁷, and R⁸; X is a bond formed as a result of a reaction between one of R² to R⁴ and one of R⁵ to R⁸ and represents an amide bond, an ester bond, or a thioester bond; and A² represents one of R⁵, R⁶, R⁷, R⁸, and a group represented by Formula (12). In Formula (12), L¹, W², and X are as defined above; and the symbol "Y²⁻⁴" means any one of Y², Y³, and Y⁴. In Formula (10), at least one of the three W²s is the group represented by Formula (11), wherein A²s, W²s, and Xs, if present plural in number, may be the same as or different from each other, respectively.

In Formula (13), L¹, L³, Y¹, Y², Y³, and Y⁴ are as defined above; and W³ represents one of R², R⁴, and a group represented by Formula (14). In Formula (14), Ring Z¹ is as defined above; the symbol "R⁵⁻⁸" represents any one of R⁵, R⁶, R⁷, and R⁸; X is a bond formed as a result of a reaction between R² or R⁴ and one of R⁵ to R⁸ and represents an amide bond, an ester bond, or a thioester bond; and A³ represents one of R⁵, R⁶, R⁷, R⁸, and a group represented by Formula (15). In Formula (15), L¹, L³, W³, and X are as defined above; and the symbol "Y^{2,4}" means one of Y² and Y⁴. In Formula (13), at least one of the two W³s is the group represented by Formula (14), wherein A³s, W³s, and Xs, if present plural in number, may be the same as or different from each other, respectively.

The prepolymers according to the present invention are soluble in a solvent. Examples of such solvents include amides such as N,N-dimethylformamide, N,N-dimethylacetamide, and N-methyl-2-pyrrolidone; sulfoxides such as dimethyl sulfoxide; sulfones; nitriles such as acetonitrile, propionitrile, and benzonitrile; ketones such as acetone, methyl ethyl ketone, diethyl ketone, methyl isobutyl ketone, cyclopentanone, and cyclohexanone; esters such as formic esters, acetic esters, propionic esters, benzoic esters, ethyl lactate, γ-butyrolactone, and propylene glycol monomethyl ether acetate (PGMEA); ethers such as dioxane, tetrahydrofuran, diethyl ether, ethylene glycol monoethyl ether, ethylene glycol diethyl ether, and propylene glycol monomethyl ether (PGME); alcohols such as methanol, ethanol, propanol, butanol, ethylene glycol, and propylene glycol; halogenated hydrocarbons such as dichloromethane, dichloroethane, chloroform, carbon tetrachloride, and chlorobenzene; nitro compounds such as nitromethane; aromatic hydrocarbons such as benzene, toluene, xylenes, ethylbenzene, and mesitylene; alicyclic hydrocarbons such as cyclohexane and methylcyclohexane; aliphatic hydrocarbons such as hexane, heptane, and octane; and mixtures of these solvents.

The prepolymers according to the present invention can be especialy easily dissolved in aprotic polar solvents such as N,N-dimethylformamide, N,N-dimethylacetamide, and N-methyl-2-pyrrolidone and have a solubility (g/100-g at 20°C) in one of the three solvents of 1 or more, preferably 5 or more, and more preferably 10 or more.

The prepolymers according to the present invention are excellent in solubility in a coating solvent. For example, when a prepolymer according to the present invention is dissolved in N,N-dimethylacetamide to yield a solution having a concentration of 15 percent by weight, and the solution is passed through a filter with a pore size of 0.2 µm, the filtration rate is about 0.02 to about 0.8 g/min.cm², and preferably about 0.1 to about 0.5 g/min.cm².

The prepolymer compositions according to the present invention are compositions comprising a solvent and the prepolymer according to the present invention dissolved in the solvent. Any solvent will do, as long as it can dissolve the prepolymer and does not adversely affect, for example, polymerization and cyclization reactions. For example, the above-mentioned solvents can be used herein.

If necessary, the prepolymer compositions according to the present invention may further comprise one or more additional components. The additional components include a monomer component used as raw materials for the prepolymers (the compound A and/or the compound B). By adding such a monomer component, the polymer chain of the prepolymer can be extended to thereby yield a crosslinked polymer having a higher molecular weight. The amount of the monomer component is, for example, about 0 to 50 parts by weight, and preferably 5 to 30 parts by weight, per 100 parts by weight of the prepolymer. Especially, the prepolymer composition preferably further comprises about 10 to about 25 parts by weight of the compound B per 100 parts by weight of the prepolymer when the compound A used as a monomer component has a plurality of (e.g., two to four) functional groups or sets of functional groups bound to the central skeleton which constitute a two-dimensional structure or three-dimensional structure, and the compound B used as a monomer component has a plurality of (e.g., two to four, preferably) functional groups or sets of functional groups bound to the central skeleton which constitute a one-dimensional structure (a linear structure) or two-dimensional structure (a chain structure at an angle).

Catalysts for accelerating, for example, polymerization or cyclization reaction can be used as additional components. Representative examples of the catalysts are acid catalysts such as sulfuric acid, methanesulfonic acid, and p-toluenesulfonic acid; and base catalysts. The amount of the catalysts is, for example, about 0 to about 10 percent by mole, and preferably about 0 to about 5 percent by mole, to the total amount of the monomer components constituting the prepolymer (the compounds A and B) and an additional monomer component, if added.

The prepolymer compositions according to the present invention may further comprise a thickening agent for increasing the viscosity of the resulting solution. Representative examples of the thickening agent are alkylene glycols such as ethylene glycol, diethylene glycol, triethylene glycol, and polyethylene glycol; and polyalkylene glycols. The amount of the thickening agent is, for example, about 0 to about 20 percent by weight, and preferably about 0 to about 10 percent by weight, based on the total amount of the compositions. The prepolymer compositions according to the present invention may further comprise one or more monocarboxylic acids for adjusting the molecular weight after polymerization, and/or one or more dicarboxylic acids for adjusting the degree of crosslinking after polymerization. Representative examples of the monocarboxylic acids include monocarboxylic acids such as adamantanecarboxylic acid and benzoic acid; and monocarboxylic acid derivatives such as adamantanecarboxylic acid methyl ester and benzoic acid methyl ester. Representative examples of the dicarboxylic acids include dicarboxylic acids such as terephthalic acid; and dicarboxylic acid derivatives such as terephthalic acid dimethyl ester. The amount of the monocarboxylic acids is, for example, about 0 to about 10 percent by mole, and preferably about 0 to about 5 percent by mole, to the total amount of the monomer components constituting the prepolymer (the compounds A and B) and an additional monomer component, if added. The amount of the dicarboxylic acids is, for example, about 0 to about 100 percent by mole, and preferably about 0 to about 50 percent by mole, to the total amount of the monomer components constituting the prepolymer (the compounds A and B) and an additional monomer component, if added.

The prepolymer compositions according to the present invention may further comprise an adhesion promoter for increasing the adhesion of a dielectric film with a substrate, which dielectric film is formed on the substrate. Representative examples of the adhesion promoter include trimethoxyvinylsilane, hexamethyldisilazane, γ-aminopropyltriethoxysilane, and aluminum monoethyl acetoacetate diisopropylate. The amount of the adhesion promoter is, for example, about 0 to about 10 percent by weight, and preferably about 0 to about 5 percent by weight, to the total amount of the monomer components constituting the prepolymer (the compounds A and B) and an additional monomer component, if added.

The prepolymer compositions according to the present invention may further comprise a component comprising one or more other polymers regardless of their structures such as straight-chain or branched-chain structure, for adjusting the physical properties of the resulting film, such as mechanical strength, thermal conductivity, and coefficient of thermal expansion, or for adjusting film properties during film formation, such as relaxed inner stress upon film formation. The prepolymer compositions may comprise, for example, a polyimidazole and/or a polyoxazole for increasing the heat resistance of the film.

The prepolymer compositions according to the present invention can be prepared by admixing the prepolymer and the solvent, and, if necessary, additives with stirring or with leaving stand to thereby dissolve the prepolymer in the solvent. The dissolution can be carried out in an atmosphere of the air, as long as the polyamine derivative is not oxidized, but is preferably carried out in an atmosphere of an inert gas such as nitrogen or argon gas. The temperature for dissolving the prepolymer can be set as appropriate according typically to the solubility of the prepolymer and the boiling point of the solvent. The dissolution of the prepolymer can be carried out with heating, as long as the prepolymer is not converted into a crosslinked polymer, and the temperature herein is, for example, about 0°C to about 200°C, and preferably about 10°C to about 150°C.

The concentration of the prepolymer in the prepolymer composition can be set as appropriate under the consideration typically of the solubility of the prepolymer, coatability, and workability, and is, for example, about 5 to about 70 percent by weight, and preferably about 10 to about 60 percent by weight. The prepolymers according to the present invention are excellent in solubility in a solvent and can thereby yield prepolymer compositions having a high concentration. Dielectric films formed from the prepolymer compositions having such a high concentration can have a larger film thickness and thereby show excellent electrical properties. Consequently, dielectric films having a variety of film thicknesses corresponding to various semiconductor production processes can be formed.

The high-molecular-weight polymers and dielectric films according to the present invention can be obtained by applying the prepolymer composition as a coating composition to a base material, and subjecting the resulting film further to reaction. More specifically, the coated film is subjected to polymerization and/or cyclization reaction as a result of heating (burning). Examples of the base material are silicon wafers, metal substrates, and ceramic substrates. The application procedure (coating procedure) is not specifically limited and can be a conventional procedure such as spin coating, dip coating, or spraying.

The heating temperature is not specifically limited, as long as it is such a temperature that can convert the prepolymer into a high-molecular-weight polymer, and is generally about 100°C to about 500°C, and preferably about 150°C to about 450°C. The heating may be carried out at a constant temperature or at temperatures with a stepwise gradient. The heating operation can be conducted in an atmosphere of the air, as long as the properties of the resulting film are not adversely affected, but it is preferably conducted in an atmosphere of an inert gas such as nitrogen or argon gas, or in a vacuum atmosphere.

As a result of the heating, the prepolymer has an increased molecular weight typically by polycondensation and thereby yields a corresponding high-molecular-weight polymer. When the prepolymer is a compound having a precursor structure of the ultimate structure, a cyclization reaction, for example, generally proceeds with an increasing molecular weight of the prepolymer, to thereby yield a high-molecular-weight polymer having a desired structure. When the prepolymer has one or more protecting groups, the increase in molecular weight and the cyclization reaction proceed generally with elimination of the protecting groups. As a result of the cyclization reaction, for example, an imidazole ring, an oxazole ring, a thiazole ring, and an imide ring are formed from corresponding precursor structures, respectively.

For example, a prepolymer represented by Formula (7), (10) or (13) can yield a high-molecular-weight polymer comprising, as main constitutional units, an adamantane ring derived from the adamantanepolycarboxylic acid derivative represented by Formula (3), (4), or (5), a monocyclic or polycyclic aromatic or nonaromatic ring derived from the polyamine derivative represented by Formula (6), and a nitrogen-containing ring formed in a polycondensed moiety. A prepolymer derived from a tetrafunctional adamantanepolycarboxylic acid derivative and represented by Formula (7) can yield a network polymer film having a multiplicity of pores and having a structure which is crosslinked in four directions with the adamantane skeleton as vertices (crosslinking points) (i.e., a unit in which three hexagons each possess two sides in common). A prepolymer derived from a trifunctional adamantanepolycarboxylic acid derivative and represented by Formula (10) can yield a highly crosslinked polymer film having a multiplicity of pores and comprising a structure which is crosslinked in three directions with the adamantane skeleton as vertices (crosslinking points) (i.e., a unit in which three hexagons each posses two vertices or two sides in common). A prepolymer derived from a bifunctional adamantanepolycarboxylic acid derivative and represented by Formula (13) can yield a porous high polymer film having a looser packing structure than a high-molecular-weight polymer obtained directly from a monomer mixture. This is because one polymer molecular chain is prevented from penetrating a region occupied by another polymer molecule due to the excluded volume effect between segments in the polymer molecular chain.

The high-molecular-weight polymers and dielectric films according to the present invention are substantially insoluble in a solvent. For example, they have a solubility (g/100-g at 20°C) in N,N-dimethylformamide, N,N-dimethylacetamide, and N-methyl-2-pyrrolidone of generally less than 1, and usually less than 0.5.

The resulting dielectric films according to the present invention have a multiplicity of uniformly dispersed molecular-scale pores inside and thereby have excellent (low) relative dielectric constants.

The film thickness of dielectric films formed as a result of heating can be set as appropriate according to the purpose and is generally about 50 nm or more (about 50 to about 2000 nm), preferably about 100 nm or more (about 100 to about 2000 nm), and more preferably about 300 nm or more (about 300 to about 2000 nm). Dielectric films having a film thickness less than 50 nm may have impaired electrical properties such as invitation of leak current or may not be sufficiently smoothed by chemical mechanical polishing (CMP) in semiconductor production processes. Accordingly, films having such an excessively small film thickness are not suitable especially as interlayer dielectric films.

In a possible procedure, a dielectric film may be formed by applying a prepared high-molecular-weight polymer (crosslinked polymer) to a substrate by coating. Such a high-molecular-weight polymer, however, has a very low solubility in a solvent and cannot significantly form a thin film by coating. In another possible procedure, a dielectric film may be formed by applying a solution of monomer components (the compounds A and B) in a solvent to a substrate. The resulting film, however, may frequently have a decreased porosity, because the monomer components come into pores of the resulting high-molecular-weight polymer, and a polymer chain formed newly penetrates pores of the high-molecular-weight polymer to clog the pores upon polymerization on the substrate. In contrast to this, the prepolymers according to the present invention are highly soluble in a solvent, can yield solutions having a high concentration, and can thereby easily form thin films having desired thicknesses. In addition, they can undergo smooth polymerization while being prevented from coming-in of monomer components into pores or penetration of polymer chains through pores, because they have such a structure that monomer components have undergo polymerization to a some extent. Consequently, they can easily constitute a highly regular porous structure having a multiplicity of uniformly dispersed molecular-scale pores inside. Dielectric films comprising the resulting high-molecular-weight polymers have high porosities, thereby show low relative dielectric constants, and have sufficient heat resistance and mechanical strength as a result of crosslinking. Additionally, they can significantly prevent copper from diffusing from interconnections.

The dielectric films according to the present invention show low dielectric constants and high heat resistance, can thereby be used as dielectric films, and are typically useful as interlayer dielectric films in electronic materials/components for, for example, semiconductor devices.

### Examples

The present invention will be illustrated in further detail with reference to several examples below which by no means limit the scope of the present invention. The filtration rates of prepolymers were determined by dissolving a sample prepolymer in N,N-dimethylacetamide to yield a solution having a concentration of 15 percent by weight, and passing the solution through a filter with a pore size of 0.2 µm. The film thicknesses of polymer films were determined using an ellipsometer. The densities of polymer films were determined by analyzing determined X-ray reflectance, and relative electric constants thereof were determined after forming an aluminum electrode on a surface of the film. The infrared absorption spectra were determined using a thin-film transmission method. The symbols "s", "m", and "w" in infrared absorption spectral data indicate the absorption intensities of wavelengths described in front of the symbols and mean "strong", "moderate", and "weak" absorptions, respectively. The weight-average molecular weights herein are values in terms of a polystyrene. The densities are values at 25°C.

### Example 1

Synthesis of prepolymer (imidazole precursor polymer) represented by following Formula (16):

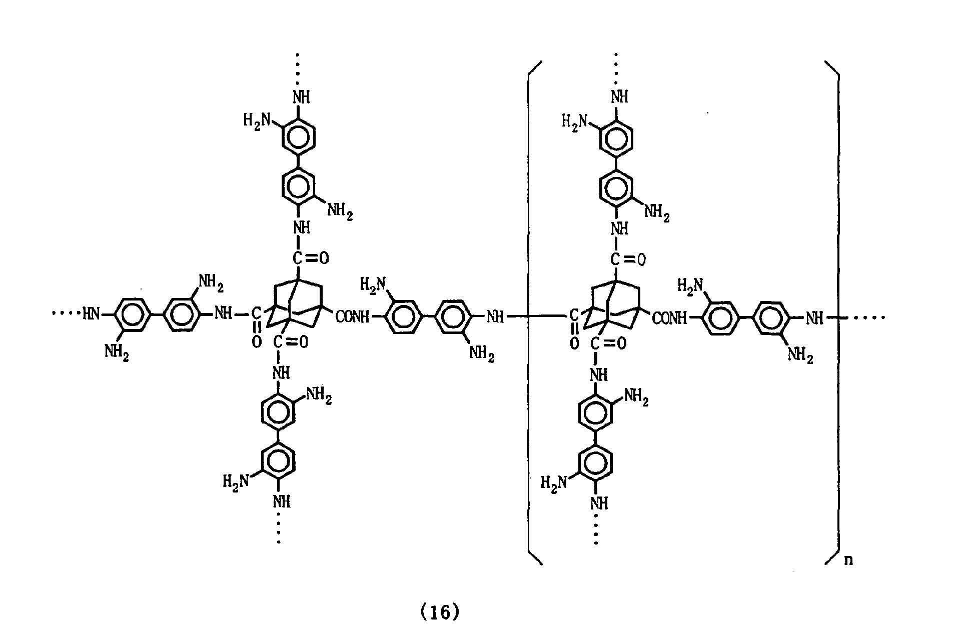

An aliquot of 3,3'-diaminobenzidine (25.4 g; 119 mmol) was placed in a three-neck flask, dissolved in 200 g of N,N-dimethylacetamide (DMAc), and purging with nitrogen was applied for thirty minutes. Under nitrogen gas flow, 3.00 g (7.8 mmol) of adamantanetetracarbonyl chloride as a 1.5 percent by weight solution in DMAc was added dropwise over one hour to the stirred solution in the flask under ice-cooling. The mixture was further stirred for one hour after the completion of the addition. The reaction mixture after the completion of reaction was placed into an eight-fold amount of water, and precipitates were separated by filtration, washed, and dried. The dried precipitates were dissolved again in DMAc to yield a solution having a solid content of 12 percent by weight, and the solution was placed in an eight-fold amount of water. The precipitates were separated by filtration, washed, dried, and thereby yielded 5.93 g of the title prepolymer in a yield of 59.3%. This has a weight-average molecular weight of about 1x 10⁴. In Formula (16), "n" is an repetition number and represents an integer of 0 or more. This prepolymer is included in the prepolymers represented by Formula (7).

### [NMR spectral data]

### ¹H-NMR (DMSO-d₆) δ: 1.51-2.60 (m, adamantane ring), 4.63 (b, NH₂), 6.45-7.58 (m, aromatic ring), 8.71-8.88 (q, - CONH-)

### Example 2

### Synthesis of a prepolymer (imidazole precursor polymer) represented by following Formula (17):

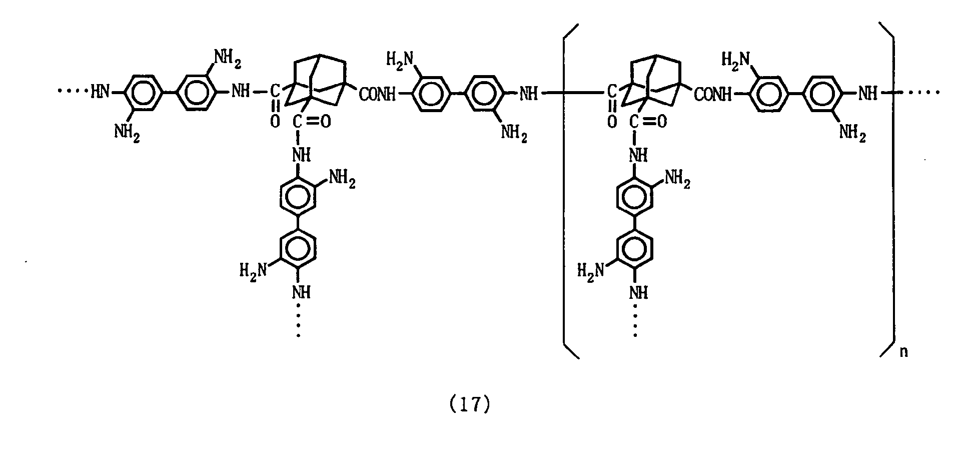

An aliquot of 3,3'-diaminobenzidine (22.3 g; 104 mmol) was placed in a three-neck flask, dissolved in 200 g of N,N-dimethylacetamide (DMAc), and purging with nitrogen was applied for thirty minutes. Under nitrogen gas flow, 3.00 g (9.3 mmol) of adamantanetricarbonyl chloride as a 3.0 percent by weight solution in DMAc was added dropwise to the stirred solution in the flask under ice-cooling over one hour. The mixture was further stirred for one hour after the completion of the addition. The reaction mixture after the completion of reaction was placed into an eight-fold amount of water, and precipitates were separated by filtration, washed, and dried. The dried precipitates were dissolved again in DMAc to yield a solution having a solid content of 12 percent by weight, and the solution was placed in an eight-fold amount of water. The precipitates were separated by filtration, washed, dried, and thereby yielded 5.45 g of the title prepolymer in a yield of 60.8%. This has a weight-average molecular weight of about 1x 10⁴. In Formula (17), "n" is an repetition number and represents an integer of 0 or more. This prepolymer is included in the prepolymers represented by Formula (10).

### [NMR spectral data]

### ¹H-NMR (DMSO-d₆) δ: 1.50-2.59 (m, adamantane ring), 4.63 (b, NH₂), 6.45-7.58 (m, aromatic ring), 8.71-8.86 (q, - CONH-)

### Example 3

Synthesis of a prepolymer (imidazole precursor polymer) represented by following Formula (18):

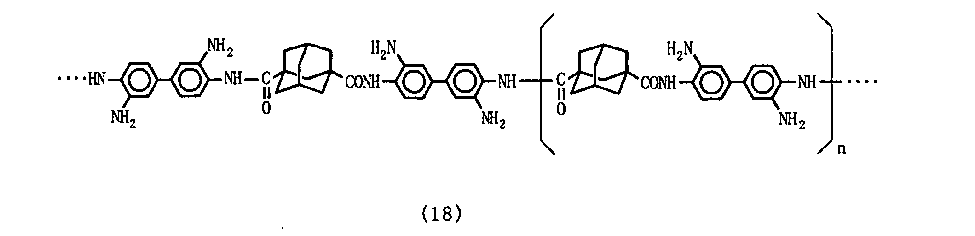

An aliquot of 3,3'-diaminobenzidine (2.27 g; 11 mmol) was placed in a three-neck flask, dissolved in 55 g of N,N-dimethylformamide (DMF), and purging with nitrogen was applied for thirty minutes. Under nitrogen gas flow, 3.00 g (11 mmol) of adamantanedicarbonyl chloride as a 3.0 percent by weight solution in DMAc was added dropwise to the stirred solution in the flask under ice-cooling over one hour. The mixture was further stirred for one hour after the completion of the addition. The reaction mixture after the completion of reaction was placed into an eight-fold amount of water, and precipitates were separated by filtration, washed, and dried. The dried precipitates were dissolved again in DMF to yield a solution having a solid content of 12 percent by weight, and the solution was placed in an eight-fold amount of water. The precipitates were separated by filtration, washed, dried, and thereby yielded 3.57 g of the title prepolymer in a yield of 55.4%. The prepolymer has a weight-average molecular weight of about 8000. In Formula (18), "n" is a repetition number and represents an integer of 0 or more. This prepolymer is included in the prepolymers represented by Formula (13).

### [NMR spectral data]

### ¹H-NMR (DMSO-d₆) δ: 1.49-2.61 (m, adamantane ring), 4.60 (b, NH₂), 6.43-7.58 (m, aromatic ring), 8.69-8.88 (q, - CONH-)

### Example 4

### Synthesis of an oxazole precursor polymer corresponding to the imidazole precursor polymer represented by Formula (16)

An aliquot of 3,3'-dihydroxybenzidine (22.3 g; 103 mmol) was placed in a three-neck flask, dissolved in 200 g of N,N-dimethylacetamide (DMAc), and purging with nitrogen was applied for thirty minutes. Under nitrogen gas flow, 3.00 g (7.8 mmol) of adamantanetetracarbonyl chloride as a 3.0 percent by weight solution in DMAc was added dropwise to the stirred solution in the flask under ice-cooling over one hour. The mixture was further stirred for one hour after the completion of the addition. The reaction mixture after the completion of reaction was placed in an eight-fold amount of methanol, and precipitates were separated by filtration, washed, and dried. The dried precipitates were dissolved again in DMAc to yield a solution having a solid content of 12 percent by weight, and the solution was placed in an eight-fold amount of methanol. The precipitates were separated by filtration, washed, dried, and thereby yielded 3.21 g of the title prepolymer in a yield of 27.5%. The prepolymer has a weight-average molecular weight of about 3x 10⁴. This prepolymer is included in the prepolymers represented by Formula (7).

### [NMR spectral data]

### ¹H-NMR (DMSO-d₆) δ: 1.50-2.49 (m, adamantane ring), 4.57 (b, NH₂), 6.60-7.88 (m, aromatic ring), 8.69-8.79 (d, - CONH-)

### Example 5

### Synthesis of an oxazole precursor polymer corresponding to the imidazole precursor polymer represented by Formula (17)

An aliquot of 3,3'-dihydroxybenzidine (22.3 g; 103 mmol) was placed in a three-neck flask, dissolved in 200 g of N,N-dimethylacetamide (DMAc), and purging with nitrogen was applied for thirty minutes. Under nitrogen gas flow, 3.00 g (9.3 mmol) of adamantanetricarbonyl chloride as a 3.0 percent by weight solution in DMAc was added dropwise to the stirred solution in the flask under ice-cooling over one hour. The mixture was further stirred for one hour after the completion of the addition. The reaction mixture after the completion of reaction was placed into an eight-fold amount of water, and precipitates were separated by filtration, washed, and dried. The dried precipitates were dissolved again in DMAc to yield a solution having a solid content of 12 percent by weight, and the solution was placed in an eight-fold amount of water. The precipitates were separated by filtration, washed, dried, and thereby yielded 5.93 g of the title prepolymer in a yield of 59.3%. The prepolymer has a weight-average molecular weight of about 1x 10⁴. This prepolymer is included in the prepolymers represented by Formula (10).

### [NMR spectral data]

### ¹H-NMR (DMSO-d₆) δ: 1.50-2.49 (m, adamantane ring), 4.57 (b, NH₂), 6.61-7.80 (m, aromatic ring), 8.69-8.79 (d, - CONH-)

### Example 6

### Synthesis of an oxazole precursor polymer corresponding to the imidazole precursor polymer represented by Formula (18)

An aliquot of 3,3'-dihydroxybenzidine (2.27 g; 11 mmol) was placed in a three-neck flask, dissolved in 55 g of N,N-dimethylformamide (DMF), and purging with nitrogen was applied for thirty minutes. Under nitrogen gas flow, 3.00g (11 mmol) of adamantanedicarbonyl chloride as a 3.0 percent by weight solution in DMF was added dropwise to the stirred solution in the flask under ice-cooling over one hour. The mixture was further stirred for one hour after the completion of the addition. The reaction mixture after the completion of reaction was placed into an eight-fold amount of water, and precipitates were separated by filtration, washed, and dried. The dried precipitates were dissolved again in DMF to yield a solution having a solid content of 12 percent by weight, and the solution was placed in an eight-fold amount of water. The precipitates were separated by filtration, washed, dried, and thereby yielded 3.23 g of the title prepolymer in a yield of 60.3%. The prepolymer has a weight-average molecular weight of about 9000. This prepolymer is included in the prepolymers represented by Formula (13).

### [NMR spectral data]

### ¹H-NMR (DMSO-d₆) δ: 1.50-2.49 (m, adamantane ring), 4.57 (b, NH₂), 6.60-7.88 (m, aromatic ring), 8.69-8.79 (d, - CONH-)

### Example 7

A coating composition was prepared by dissolving 3.00 g of the prepolymer (imidazole precursor polymer) prepared according to Example 1 and 0.552 g of 1,3,5,7-adamantanetetracarboxylic acid in 20.13 g of N,N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzimidazole film was formed. The film has a film thickness of 298 nm, a density of 1.05 g/cm³, and a relative dielectric constant of 2.3. The constitutive prepolymer was found to have a filtration rate of 0.12 g/min. cm².

### [Infrared absorption spectral data (cm⁻¹)]

### 806 (s), 1279 (m), 1406 (m), 1450 (s), 1517 (w), 1623 (w), 2854 (s), 2904 (s), 3419 (w)

### Example 8

A coating composition was prepared by dissolving 3.00 g of the prepolymer (imidazole precursor polymer) prepared according to Example 2 and 0.378 g of 1,3,5-adamantanetricarboxylic acid in 19.14 g of N,N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzimidazole film was formed. The film has a film thickness of 305 nm, a density of 1.1 g/cm³, and a relative dielectric constant of 2.4. The constitutive prepolymer was found to have a filtration rate of 0.18 g/min. cm².

### [Infrared absorption spectral data (cm⁻¹)]

### 805 (s), 1279 (m), 1407 (m), 1450 (s), 1517 (w), 1623 (w), 2852 (s), 2904 (s), 3419 (w)

### Example 9

A coating composition was prepared by dissolving 3.01 g of the prepolymer (imidazole precursor polymer) prepared according to Example 3 in 16.99 g of N, N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzimidazole film was formed. The film has a film thickness of 320 nm, a density of 1.18 g/cm³, and a relative dielectric constant of 2.5. The constitutive prepolymer was found to have a filtration rate of 0.19 g/min.cm².

### [Infrared absorption spectral data (cm⁻¹)]

806 (s), 1278 (m), 1405 (m), 1450 (s), 1522 (w), 1627 (w), 2852 (s), 2906 (s), 3418 (w)

### Example 10

A coating composition was prepared by dissolving 3.00 g of the prepolymer (oxazole precursor polymer) prepared according to Example 4 and 0.47 g of 1,3,5,7-adamantanetetracarboxylic acid in 19.67 g of N,N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzoxazole film was formed. The film has a film thickness of 295 nm, a density of 1.15 g/cm³, and a relative dielectric constant of 2.4. The constitutive prepolymer was found to have a filtration rate of 0.12 g/min. cm².

### [Infrared absorption spectral data (cm⁻¹)]

### 835 (m), 1280 (m), 1403 (m), 1450 (s), 1522 (w), 1625 (w), 2832 (s), 2928 (s), 3149 (w)

### Example 11

A coating composition was prepared by dissolving 3.00 g of the prepolymer (oxazole precursor polymer) prepared according to Example 5 and 0.35 g of 1,3,5-adamantanetricarboxylic acid in 19.12 g of N,N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzoxazole film was formed. The film has a film thickness of 311 nm, a density of 1.16 g/cm³, and a relative dielectric constant of 2.5. The constitutive prepolymer was found to have a filtration rate of 0.11 g/min.cm².

### [Infrared absorption spectral data (cm⁻¹)]

### 835 (m), 1280 (m), 1403 (m), 1450 (s), 1522 (w), 1625 (w), 2832 (s), 2928 (s), 3149 (w)

### Example 12

A coating composition was prepared by dissolving 3.03 g of the prepolymer (oxazole precursor polymer) prepared according to Example 6 in 16.98 g of N, N-dimethylacetamide (DMAc). The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzoxazole film was formed. The film has a film thickness of 303 nm, a density of 1.17 g/cm³, and a relative dielectric constant of 2.5. The constitutive prepolymer was found to have a filtration rate of 0.19 g/min.cm².

### [Infrared absorption spectral data (cm⁻¹)]

835 (m), 1280 (m), 1403 (m), 1451 (s), 1522 (w), 1617 (w), 2839 (s), 2925 (s), 3149 (w)

### Comparative Example 1

A coating composition having a concentration of 25 percent by weight was prepared by dissolving 5.37 g (20 mmol) of 1,3,5-adamantanetricarboxylic acid and 6.43 g (30 mmol) of 3,3'-diaminobenzidine in N-methyl-2-pyrrolidone (NMP) at room temperature in a nitrogen atmosphere. The coating composition was passed through a filter with a pore size of 0.2 µm and applied to an eight-inch silicon wafer by spin coating. This was heated at 300°C in a nitrogen atmosphere for thirty minutes, further heated at 400°C for thirty minutes, and thereby yielded a film. The infrared absorption spectrum of the polymer film was determined to find that a target crosslinked polybenzimidazole film was formed. The film has a film thickness of 418 nm, a density of 1.28 g/cm³, and a relative dielectric constant of 2.7.

### [Infrared absorption spectral data (cm⁻¹)]

### 805 (m), 1280 (m), 1403 (m), 1450 (s), 1522 (w), 1625 (w), 2857 (s), 2928 (s), 3419 (w)

### Comparative Example 2

In a flask equipped with a stirrer and a condenser were placed 5.37 g (20 mmol) of 1,3,5-adamantanetricarboxylic acid, 6.43 g (30 mmol) of 3,3'-diaminobenzidine, and 100 g of polyphosphoric acid, and the mixture was heated with stirring at 200°C in a nitrogen atmosphere for twelve hours. After cooling, the reaction mixture was combined with water, the precipitated solid was collected by filtration, washed with an aqueous sodium hydrogen carbonate solution, water, and methanol, and thereby yielded a polybenzimidazole as a solid. An attempt was made to dissolve the solid polybenzimidazole in N-methylpyrrolidone (NMP: solvent) but ended in fail. The polybenzimidazole could not be formed into a thin film by spin coating, and the target thin film was not obtained.

## Claims

1. A prepolymer as a reaction product between a compound A and a compound B, the compound A and the compound B each having two or more functional groups or sets of functional groups in one molecule, and the compound A and the compound B being capable of undergoing polymerization as a result of binding of the functional groups or sets of functional groups of one compound with the functional groups or sets of functional groups of the other compound to thereby form a high-molecular-weight polymer with a porous structure.

2. The prepolymer according to claim 1, which has a weight-average molecular weight of 200 to 100000.

3. The prepolymer according to one of claims 1 and 2, as a reaction product of a compound A and a compound B, the compound A and the compound B being capable of forming a high-molecular-weight polymer with a porous structure as a result of a polymerization process, the polymerization process comprising a first reaction process of allowing the functional groups or sets of functional groups of one compound to react with the functional groups or sets of functional groups of the other compound to form a chain bond; and a second reaction process of forming a ring at the binding site, wherein the prepolymer is a reaction product of the first reaction process.

4. The prepolymer according to claim 3, wherein the chain bond formed in the first reaction process is one of an amide bond, an ester bond, and a thioester bond, and the ring which will be formed in the second reaction process is one of an imidazole ring, an oxazole ring, a thiazole ring, and an imide ring, in the reaction between the functional groups or sets of functional groups of the compound A and the functional groups or sets of functional groups of the compound B.

5. The prepolymer according to claim 4, wherein the functional groups or sets of functional groups of the compound A are each a carboxyl group or an amino group, and wherein the functional groups or sets of functional groups of the compound B are two amino groups; an amino group and a hydroxyl group; an amino group and a mercapto group; or two carboxyl groups.

6. The prepolymer according to any one of claims 1 to 5, wherein the compound A is a compound represented by following Formula (1): 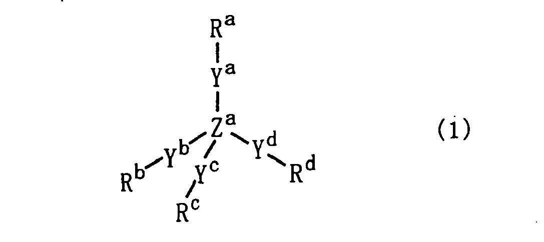 wherein Z^{a} represents a quadrivalent organic group; R^{a}, R^{b}, R^{c}, and R^{d} are the same as or different from one another and each represent a protected or unprotected carboxyl group, a haloformyl group, a protected or unprotected amino group, a protected or unprotected hydroxyl group, a protected or unprotected mercapto group, a hydrogen atom, or a hydrocarbon group; and Y^{a}, Y^{b}, Y^{c}, and Y^{d} are the same as or different from one another and each represent a single bond or a bivalent aromatic or nonaromatic cyclic group, wherein at least two of R^{a}, R^{b}, R^{c}, and R^{d} each represent a protected carboxyl group, a haloformyl group, a protected or unprotected amino group, a protected or unprotected hydroxyl group, or a protected or unprotected mercapto group.

7. The prepolymer according to any one of claims 1 to 5, wherein the compound B is a compound represented by following Formula (2): 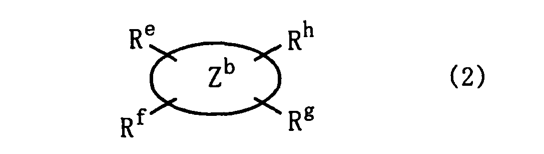 wherein Ring Z^{b} represents a monocyclic or polycyclic aromatic or nonaromatic ring; and R^{e}, R^{f}, R^{g}, and R^{h} are substituents bound to Ring Z^{b}, are the same as or different from one another, and each represent a protected or unprotected amino group, a protected or unprotected hydroxyl group, a protected or unprotected mercapto group, or a protected or unprotected carboxyl group.

8. The prepolymer according to any one of claims 1 to 3, which is a reaction product between at least one adamantanepolycarboxylic acid derivative represented by following Formula (3), (4), or (5): 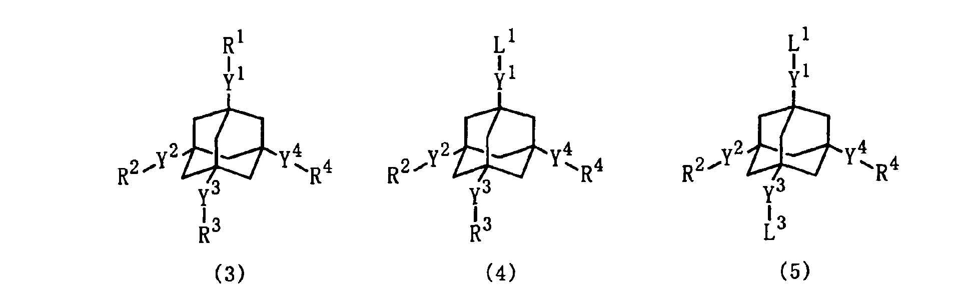 wherein R¹, R², R³, and R⁴ are the same as or different from one another and each represent a protected or unprotected carboxyl group or a haloformyl group; Y¹, Y², Y³, and Y⁴ are the same as or different from one another and each represent a single bond or a bivalent aromatic or nonaromatic cyclic group; and L¹ and L³ are the same as or different from each other and each represent a hydrogen atom, a protected or unprotected carboxyl group, or a hydrocarbon group, and a polyamine derivative represented by following Formula (6): 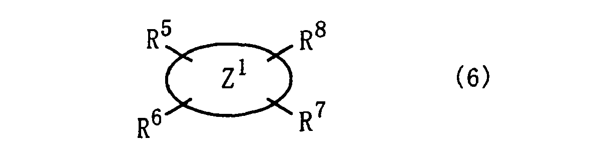 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer comprises an amide bond, an ester bond, or a thioester bond formed as a result of a reaction of the protected or unprotected carboxyl group or haloformyl group in R¹, R², R³, and R⁴ of the adamantanepolycarboxylic acid derivative represented by Formula (3), (4), or (5) with the protected or unprotected amino group, protected or unprotected hydroxyl group, or protected or unprotected mercapto group in R⁵, R⁶, R⁷, and R⁸ of the polyamine derivative represented by Formula (6).

9. The prepolymer according to claim 8, as a reaction product between an adamantanepolycarboxylic acid derivative represented by following Formula (3): 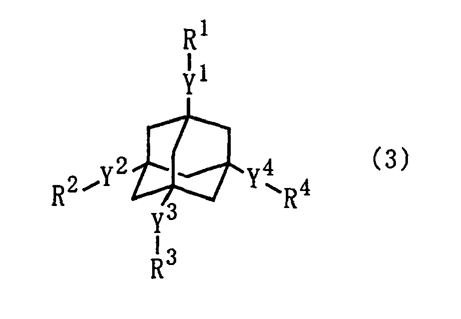 wherein R¹, R², R³, and R⁴ are the same as or different from one another and each represent a protected or unprotected carboxyl group or a haloformyl group; and Y¹, Y², Y³, and Y⁴ are the same as or different from one another and each represent a single bond or a bivalent aromatic or nonaromatic cyclic group,
and a polyamine derivative represented by following Formula (6): 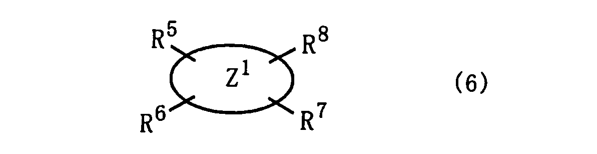 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (7): 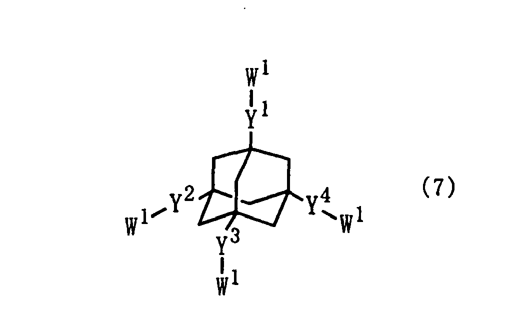 wherein Y¹, Y², Y³, and Y⁴ are as defined above; and W¹s each represent one of R¹, R², R³, R⁴, and a group represented by following Formula (8): 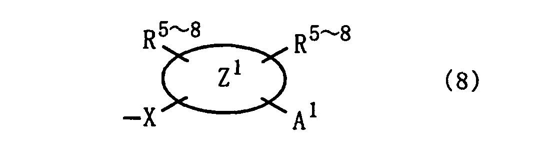 wherein Ring Z¹ is as defined above; R⁵⁻⁸ represents any one of R⁵, R⁶, R⁷, and R⁸; X is a bond formed as a result of a reaction between one of R¹ to R⁴ and one of R⁵ to R⁸ and represents an amide bond, an ester bond, or a thioester bond; and A¹ represents one of R⁵, R⁶, R⁷, R⁸, and a group represented by following Formula (9): 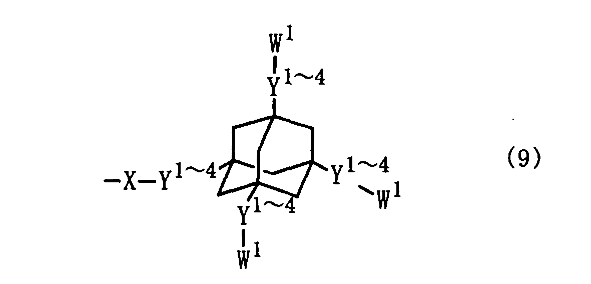 wherein W¹s and X are as defined above; and Y¹⁻⁴ represents any one of Y¹, Y², Y³, and Y⁴,
wherein at least one of the four W¹s in Formula (7) is the group represented by Formula (8), and wherein A¹s, W¹s, and Xs, if present plural in number, may be the same as or different from each other, respectively.

10. The prepolymer according to claim 8, as a reaction product between an adamantanepolycarboxylic acid derivative represented by following Formula (4): 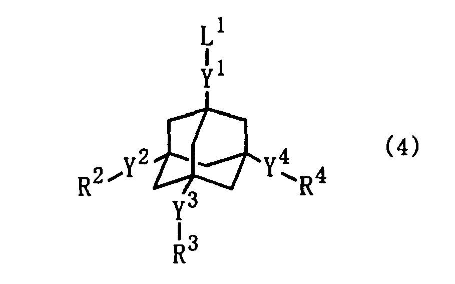 wherein L¹ represents a hydrogen atom, a protected or unprotected carboxyl group, or a hydrocarbon group; R², R³, and R⁴ are the same as or different from one another and each represent a protected or unprotected carboxyl group or a haloformyl group; and Y¹, Y², Y³, and Y⁴ are the same as or different from one another and each represent a single bond or a bivalent aromatic or nonaromatic cyclic group, and a polyamine derivative represented by following Formula (6): 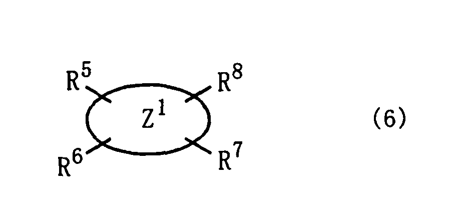 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (10): 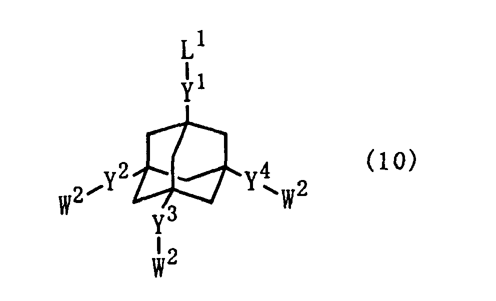 wherein L¹, Y¹, Y², Y³, and Y⁴ are as defined above; and W²s each represent one of R², R³, R⁴, and a group represented by following Formula (11): 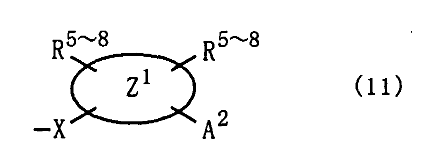 wherein Ring Z¹ is as defined above; R⁵⁻⁸ represents any one of R⁵, R⁶, R⁷, and R⁸; X is a bond formed as a result of a reaction between one of R² to R⁴ and one of R⁵ to R⁸ and represents an amide bond, an ester bond, or a thioester bond; and A² represents one of R⁵, R⁶, R⁷, R⁸, and a group represented by following Formula (12): 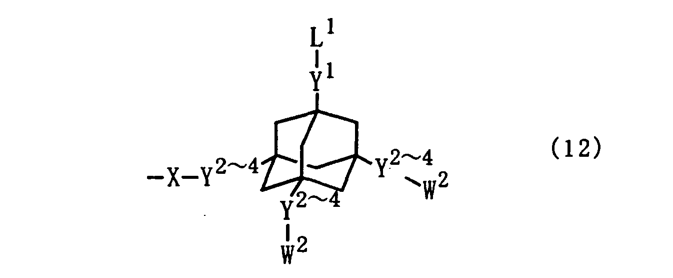 wherein L¹, W²s, and X are as defined above; and Y²⁻⁴ represents any one of Y², Y³, and Y⁴,
wherein at least one of the three W²s in Formula (10) is the group represented by Formula (11), and wherein A²s, W²s, and Xs, if present plural in number, may be the same as or different from each other, respectively.

11. The prepolymer according to claim 8, as a reaction product between an adamantanepolycarboxylic acid derivative represented by following Formula (5): 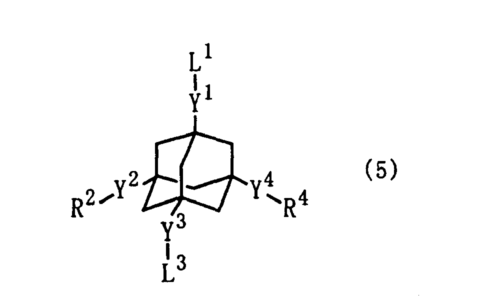 wherein L¹ and L³ are the same as or different from each other and each represent a hydrogen atom, a protected or unprotected carboxyl group, or a hydrocarbon group; R² and R⁴ are the same as or different from each other and each represent a protected or unprotected carboxyl group or a haloformyl group; and Y¹, Y², Y³, and Y⁴ are the same as or different from one another and each represent a single bond or a bivalent aromatic or nonaromatic cyclic group,
and a polyamine derivative represented by following Formula (6): 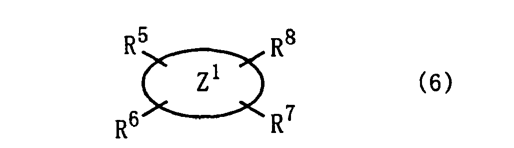 wherein Ring Z¹ represents a monocyclic or polycyclic aromatic or nonaromatic ring; and R⁵, R⁶, R⁷, and R⁸ are substituents bound to Ring Z¹, are the same as or different from one another, and each represent a protected or unprotected amino group, a protected or unprotected hydroxyl group, or a protected or unprotected mercapto group, wherein at least two of R⁵, R⁶, R⁷, and R⁸ are each a protected or unprotected amino group,
wherein the prepolymer is a compound represented by following Formula (13): 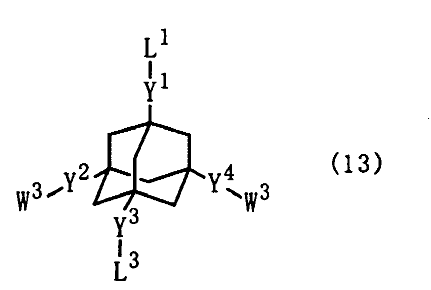 wherein L¹, L³, Y¹, Y², Y³, and Y⁴ are as defined above; and W³s each represent one of R², R⁴, and a group represented by following Formula (14): 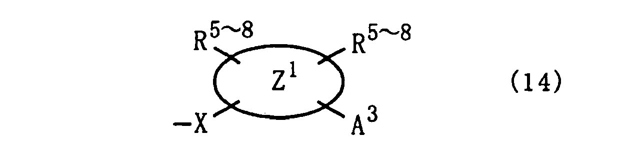 wherein Ring Z¹ is as defined above; R⁵⁻⁸ represents any one of R⁵, R⁶, R⁷, and R⁸; X is a bond formed as a result of a reaction between one of R² and R⁴, and one of R⁵ to R⁸ and represents an amide bond, an ester bond, or a thioester bond; and A³ represents one of R⁵, R⁶, R⁷, R⁸, and a group represented by following Formula (15): 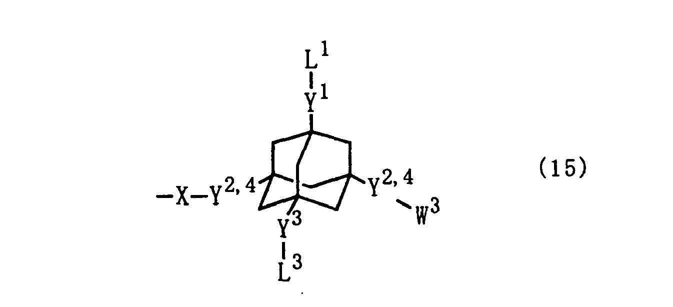 wherein L¹, L³, W³, and X are as defined above; and Y^{2,4} represents one of Y² and Y⁴,
wherein at least one of the two W³s in Formula (13) is the group represented by Formula (14), and wherein A³s, W³s, and Xs, if present plural in number, may be the same as or different from each other, respectively.

12. A prepolymer composition, as a solution of the prepolymer of any one of claims 1 to 11 in a solvent.

13. A high-molecular-weight polymer with a porous structure, as a reaction product of the prepolymer of any one of claims 1 to 11.

14. A dielectric film comprising the high-molecular-weight polymer with a porous structure of claim 13.

15. A method for producing a dielectric film, comprising the steps of applying a prepolymer composition to a base material, the prepolymer composition being a solution of the prepolymer of any one of claims 1 to 11 in a solvent; and subjecting the prepolymer composition on the base material to reaction to thereby yield a dielectric film comprising a high-molecular-weight polymer with a porous structure.
